(19) Europäisches Patentamt

European Patent Office

Office européen des brevets

(11) EP 1 731 168 A1

(12) EUROPEAN PATENT APPLICATION
published in accordance with Art. 158(3) EPC

(43) Date of publication:
13.12.2006 Bulletin 2006/50

(51) Int Cl.:
A61K 39/395 (2006.01)   A61K 47/48 (2006.01)
A61P 3/00 (2006.01)     A61P 5/02 (2006.01)
A61P 5/06 (2006.01)     A61P 5/18 (2006.01)
A61P 5/48 (2006.01)     A61P 7/00 (2006.01)
A61P 9/00 (2006.01)     A61P 15/00 (2006.01)
A61P 15/10 (2006.01)    A61P 15/18 (2006.01)
A61P 19/08 (2006.01)    A61P 25/00 (2006.01)
A61P 31/00 (2006.01)    A61P 35/00 (2006.01)
A61P 37/04 (2006.01)

(21) Application number: 05721715.0

(22) Date of filing: 29.03.2005

(86) International application number:
PCT/JP2005/006576

(87) International publication number:
WO 2005/094881 (13.10.2005 Gazette 2005/41)

(84) Designated Contracting States:
AT BE BG CH CY CZ DE DK EE ES FI FR GB GR
HU IE IS IT LI LT LU MC NL PL PT RO SE SI SK TR

(30) Priority: 30.03.2004  JP 2004098595

(71) Applicant: Takeda Pharmaceutical Company
Limited
Osaka-shi,
Osaka 541-0045 (JP)

(72) Inventors:
• INOOKA, Hiroshi;
c/o TAKEDA PHARMA.COMPANY LTD.;
Osaka-shi, Osaka 5328686 (JP)

• SUZUKI, Nobuhiro;
c/o TAKEDA PHARMA.COMPANY LTD.;
Ibaraki 300-4293 (JP)
• KOKUBO, Toshio;
c/o TAKEDA PHARMA.COMPANY LTD.;
Osaka-shi, Osaka 5328686 (JP)
• KUROKAWA, Tomofumi;
c/o TAKEDA PHARMA.COMPANY LTD;
Osaka-shi, Osaka 5328686 (JP)

(74) Representative: Kalhammer, Georg et al
Lederer & Keller
Patentanwälte
Prinzregentenstrasse 16
80538 München (DE)

(54) ANTIBODY DRUG

(57)    The present invention provides an agent for improving the blood stability of an endogenous ligand, which comprises an antibody that has affinity to the mammalian endogenous ligand but substantially does not neutralize the same, and the above-described agent for the prophylaxis and/or treatment for a disease for which an increase in the blood concentration of the endogenous ligand and/or an prolonged blood half-life is prophylactically or therapeutically effective. Provided that the above-described agent is administered alone to a mammal without co-administering the same or substantially the same compound as the endogenous ligand, the blood stability of the endogenous ligand increases and the receptor activity-regulatory action thereof is enhanced.

EP 1 731 168 A1

## Description

## Technical Field

The present invention relates to a novel pharmaceutical use of an antibody. More specifically, the present invention relates to a use of an antibody that has affinity for an endogenous ligand but does not completely neutralize the same, for enhancing the receptor activation action of the ligand by improving the stability of the ligand in blood.

## Background Art

Antibodies have excellent properties for pharmaceutical products such as low incidence of side effects and long sustainability of efficacy because of their high specificity and long half-life in blood. The first antibody medicine in the modern sense that targets a disease-specific antigen was a mouse anti-CD3 monoclonal antibody approved as a therapeutic agent for acute rejection in the United States in 1986 (trade name: Orthoclone); since then, however, development of antibody medicines had long been at a standstill because mouse monoclonal antibodies have drawbacks, for example, when they are administered to humans, anti-mouse immunoglobulin (Ig) human antibodies known as HAMA (Human Anti-Mouse Antibodies), are produced and cause efficacy reductions due to the shortened half-life of the drug and anaphylactic symptoms, and when antibody-dependent cell-mediated cytotoxicity (ADCC) or complement-dependent cytotoxicity (CDC) is utilized, the action remarkably decreases in the human body in the case of mouse Fc.

However, in the 1990s, as production technologies for chimeric antibodies, humanized antibodies, and fully human antibodies were established and the problem of immunogenicity and efficacy reductions was resolved, antibody medicines became highlighted again, and therapeutic drugs mainly for intractable diseases such as cancers, graft rejection, cardiovascular diseases, infectious diseases, and autoimmune diseases were brought into actual application one by one; more than 100 antibody medicines, including those in the clinical stage, have been developed to date.

Most of the conventional antibody medicines, including those under development, are based on any of the modes of action: (1) binding to the target antigen and inhibiting (namely, neutralizing) the function thereof [e.g., anti-TNFα antibody (trade name: Remicade), anti-RSV surface protein antibody (trade name: Synagis)]; (2) functioning as a carrier for delivering a drug (for example, chemotherapeutic agent, toxin, radioisotope, cytokine, etc.) to pathogenic cells that express the target antigen [e.g., $^{90}$Y-bound anti-CD20 antibody (trade name: Zevalin), calicheamycin-bound anti-CD33 antibody (trade name: Mylotarg)]; and (3) attacking the cells that express the target antigen by utilizing ADCC or CDC [e.g., anti-HER2 antibody (trade name: Herceptin), anti-CD20 antibody (trade name: Rituxan)].

By contrast, as a new generation of antibody medicines, development of what are called agonist antibodies, which are intended to enhance the function of the target antigen, rather than neutralizing the target antigen or killing the antigen-expressing cells, is being considered. For example, there has been proposed a method comprising cross-linking monomer molecules of a receptor that forms an oligomer by its ligand binding to activate signal transduction, like a tyrosine kinase type receptor, by using an antibody against such monomer molecules to activate the receptor (see, for example, WO 01/79494).

However, with regard to antibody medicines that target humoral biologically active peptides (proteins) serving as ligand molecules for receptors on the cell surface, such as cytokines and growth factors, neutralizing antibodies that inhibit the function of the target antigen (i.e., receptor activation action) are mainly utilized.

Although biologically active peptides (proteins) are utilized as pharmaceuticals since they are ligand molecules that exhibit various pharmacological actions in a living organism, these are highly susceptible to peptidases (proteases) and are likely to undergo renal clearance because of their small molecular weights so that their active forms generally have short half-life in the living organism. For this reason, the biological active peptides (proteins) have a drawback in terms of efficacy sustainability and a frequent dosing is often necessary. Although there have been attempts to extend the blood half-life of a biologically active protein such as a cytokine to improve the efficacy sustainability by using in combination an antibody that binds to the protein at a site where the protein's biological activity is not affected, in administering a pharmaceutical with the protein as an active ingredient (see, for example, Japanese Patent Kohyo publication No. SHO-60-502104), no such approaches have been in actual application.

Also, unlike other biotechnology-based pharmaceutical products such as cytokines, which exhibit their efficacy at low doses, clinical doses of conventional antibody medicines are generally enormous, in some cases, are as much as several hundred milligrams. Currently, antibody medicines are produced as recombinants using animal cells such as CHO cells, and are very expensive due to limitations on the amount of antibody produced and culture scale; therefore, the indications of antibody medicines are now limited to intractable diseases for which no adequate therapeutic effect is obtained with any existing therapeutic drug. Although antibody medicines under clinical development include therapeutic drugs for chronic diseases affecting many patients (e.g., rheumatoid arthritis), it is critical to overcome the cost-related problems before the antibody medicine market is expanded to cover the area of lifestyle-related diseases such as diabetes and hyperlipidemia.

Furthermore, it is also important from the viewpoint of safety to reduce the dose of an antibody medicine. The majority of antibody medicines under development are either chimeric or humanized antibodies, however, they sometimes cause production of an anti-Ig human antibody (HACA or HAHA) because these comprise a mouse-derived variable region or CDR. Although the problem of anti-Ig human antibody induction is nearly completely resolved by establishing a technology for producing a fully human antibody, the possibility of production of anti-human Ig human antibody with administration of an antibody in large amounts cannot be ruled out because any antibody has the property of being non-self to the host as the intrinsic fate thereof.

**Disclosure of the Invention**

It is an object of the present invention to provide a new generation of antibody medicine based on a different basic concept from that of the conventional antibody medicines. Particularly, the present invention is directed to providing a novel antibody medicine that is useful as a therapeutic drug in the area of diseases for which existing antibody medicines do not have a sufficient therapeutic effect and the market scale is large, such as life style-related diseases. In another aspect, the present invention is directed to reducing the amount of antibody used per course of treatment by providing an antibody medicine capable of having a therapeutic effect at much lower doses compared with conventional antibody medicines, to thereby contribute to medical economics.

The present invention is partially based on the finding that an anti-PACAP monoclonal antibody recognizing a certain partial sequence of pituitary adenylate cyclase-activating polypeptide (PACAP) as the antigenic determinant does not inhibit the action of PACAP [adenylate cyclase (AC) activation action], and that when the anti-PACAP antibody is administered alone to a mouse, endogenous PACAP which is normally not detected in blood, is detected as a complex with the antibody (i.e., stabilized in blood). Based on this finding, the present inventors thought that by administering an antibody that has affinity for an endogenous ligand of the recipient animal and does not completely inhibit the action of the ligand (non-neutralizing antibody) alone to the animal (i.e., without administering the ligand or an equivalent thereof), it would be possible to improve the blood stability thereof (i.e., increase the ligand concentration in blood) while retaining the physiological action of the endogenous ligand, and, as a result, to enhance the receptor activation action of the ligand. Hence, the present inventors newly prepared a non-neutralizing antibody against glucagon-like peptide-1 (GLP-1), succeeded in raising the plasma GLP-1 concentration to a level showing efficacy by administering this to a rat, and developed the present invention.

Accordingly, the present invention relates to

[1] an agent for improving the blood stability of a mammalian endogenous ligand, which comprises an antibody that has an affinity to the endogenous ligand but does not neutralize the same substantially,

[2] the agent of [1] above, wherein the improved blood stability of the endogenous ligand results in the enhancement of receptor activity-regulatory action thereof,

[3] the agent of [1] above, wherein the neutralizing activity of the antibody is about 80% or less,

[4] the agent of [1] above, wherein the blood concentration of the endogenous ligand becomes about twice or more compared to the case where the antibody is not administered,

[5] the agent of [1] above, wherein the blood half-life of the complex of the endogenous ligand and the antibody is about twice or more as that of the endogenous ligand alone,

[6] the agent of [1] above, wherein the blood half-life of the free endogenous ligand is about one week or less,

[7] the agent of [1] above, wherein the endogenous ligand is a peptidic compound,

[8] the agent of [7] above, wherein the endogenous ligand is one against a G protein-coupled receptor,

[9] the agent of [8] above, wherein the endogenous ligand is one belonging to secretin/glucagon super family,

[10] the agent of [9] above, wherein the endogenous ligand is selected from the group consisting of GLP-1, calcitonin, PACAP, VIP and analogs thereof,

[11] the agent of [8] above, wherein the endogenous ligand is selected from the group consisting of LHRH, metastin, GPR7/GPR8 ligand, MSH, ghrelin, apelin and analogs thereof,

[12] the agent of [7] above, wherein the endogenous ligand is selected from the group consisting of EPO, TPO, insulin, interferon, growth hormone, GM-CSF, leptin, adiponectin and analogs thereof,

[13] the agent of [7] above, wherein the endogenous ligand is selected from the group consisting of ANP, BNP, CNP, betacellulin, betacellulin-$\delta$4, adrenomedullin and analogs thereof,

[14] the agent of [1] above, which is for the prophylaxis and/or treatment of a disease in which an increased blood concentration and/or a prolonged blood half-life of the endogenous ligand are/is effective for the prophylaxis and/or treatment thereof,

[15] the agent of [14] above, wherein the disease is selected from the group consisting of metabolic disease, bone and joint disease, cardiovascular disease, cranial nerve disease, infectious disease, cancer, blood disorder, urologic disease, infertility/erectile dysfunction, deficient growth and immunodeficiency,

[16] a method for the prophylaxis and/or treatment of a disease in a mammal, wherein an increased blood concentration and/or a prolonged blood half-life of an endogenous ligand are/is effective for the prophylaxis and/or treatment of the disease, which method comprises administering to the mammal an effective amount of an antibody that has an affinity to the endogenous ligand but does not neutralize the same substantially, without administering a compound the same as or substantially the same as the endogenous ligand, so as to increase the blood stability of the endogenous ligand, thereby enhancing a receptor activity-regulatory action of the ligand, and

[17] a use of an antibody that has an affinity for an endogenous ligand but does not neutralize the same substantially for the manufacture of an agent for the prophylaxis and/or treatment of a disease in which an increased blood concentration and/or a prolonged blood half-life of the endogenous ligand are/is effective for the prophylaxis and/or treatment thereof, and the like.

When administered to an animal, the antibody of the present invention is capable of binding to an endogenous ligand and stabilizing the same to raise the blood ligand concentration and, as a result, to enhance the receptor activity-regulatory action of the ligand because of its characteristic of having affinity for an endogenous ligands but not completely neutralizing the same. Also, because the antibody of the present invention exhibits its effect in an amount sufficient to capture a ligand that is present essentially only in trace amounts in blood, it permits a remarkable reduction in clinical doses compared with existing antibody medicines and enables the provision of a safer and less expensive preparation.

**Brief Description of the Drawings**

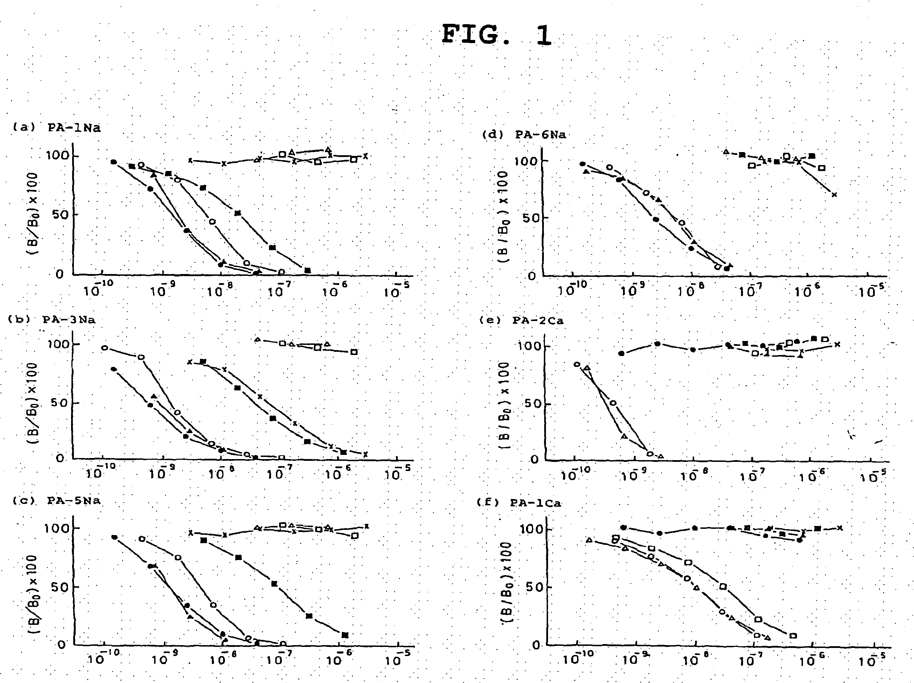

FIG. 1 shows the binding activities of various anti-PACAP monoclonal antibodies [(a) PA-1Na; (b) PA-3Na; (c) PA-5Na; (d) PA-6Na; (e) PA-2Ca; (f) PA-1Ca] against PACAP38, partial peptides thereof, and VIP. The ordinate indicates [measured amount of label (B)] /[amount of label without addition of competitive peptide (B$_0$)]$\times$100 (%); the abscissa indicates the concentration (M) of each competitive peptide. -O-: PACAP38NH$_2$; -●-: PACAP27NH$_2$; -▲-: PACAP (4-27)OH; -■-: PACAP (1-13) OH; -×-: VIP; -△-: PACAP(14-38)NH$_2$; -□-: PACAP(31-38)NH$_2$

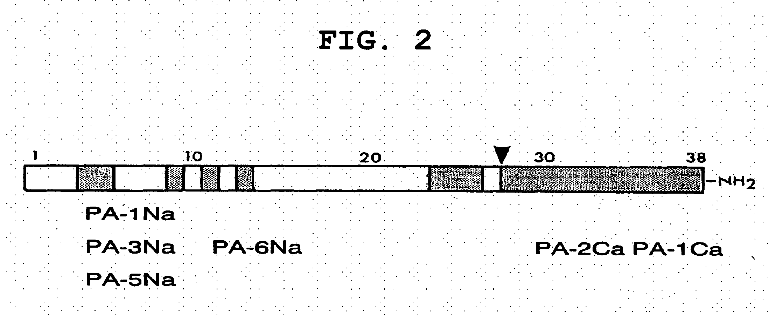

FIG. 2 is a schematic diagram showing the antigen recognition sites of various anti-PACAP monoclonal antibodies. The upper box indicates PACAP38NH$_2$, and the numerical figures thereon show amino acid numbers. The arrowhead indicates the processing site of PACAP27. The white portions of the box indicate the amino acid sequences common to VIP, and the hatched portions indicates the amino acid sequences differing from VIP.

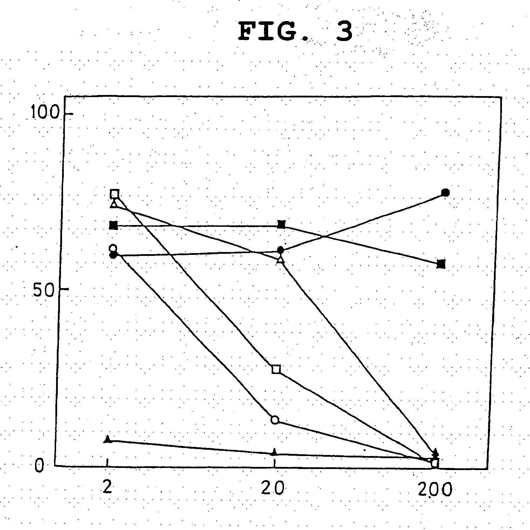

FIG. 3 shows the neutralizing activities of various anti-PACAP monoclonal antibodies on PACAP38NH$_2$. The ordinate indicates cAMP concentration (pmol/10$^5$ cells); the abscissa indicates antibody concentration (nM). -O-: PA-1Na; -△-. PA-3Na; -□-: PA-5Na; -■-: PA-6Na; -▲-: PA-2Ca; -●-: PA-1Ca

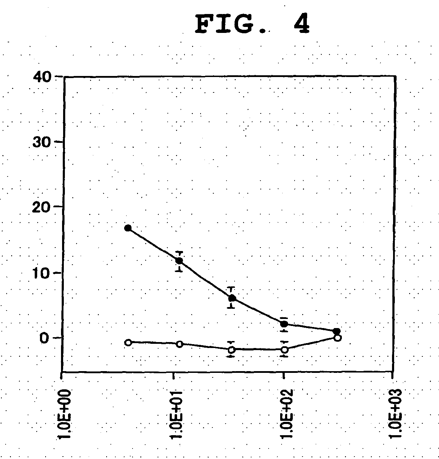

FIG. 4 shows the suppressive effect of the anti-PACAP non-neutralizing monoclonal antibody PA-6Na on PACAP38 degradation by DPP-IV. The ordinate indicates the degree of binding of $^{125}$I-PACAP27 to PACAP receptor (the residual radioactivity difference between experiment 1 and experiment 2 is shown as %, with the residual radioactivity measured with the receptor binding site saturated with excess PACAP38 as 0%, and the activity measured in the absence of PACAP38 as 100%); the abscissa indicates the dilution factor of DPP-IV expressing cell membrane fraction. -O-: in the presence of PA-6Na; -●-: in the absence of PA-6Na

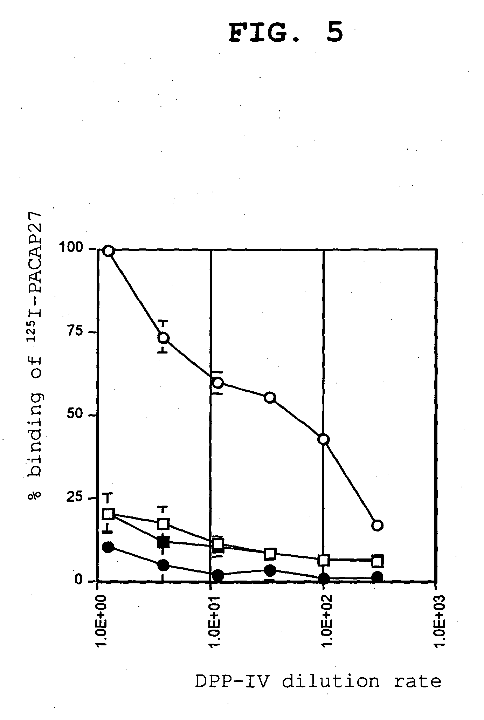

FIG. 5 shows the suppressive effect of the anti-PACAP non-neutralizing monoclonal antibody (PA-6Na) on PACAP38 degradation by recombinant DPP-IV. The ordinate indicates the degree of binding of $^{125}$I-PACAP27 to PACAP receptor (ratio of residual radioactivity, with the radioactivity of input $^{125}$I-PACAP27 as 100%); the abscissa indicates the dilution factor of DPP-IV. Symbols show respective conditions: -□-: in the presence of PA-6Na and in the absence of DPP-IV inhibitor; -■-: in the co-presence of PA-6Na and DPP-IV inhibitor; -O-: in the absence of antibody and DPP-IV inhibitor; -•-: in the absence of antibody and in the presence of DPP-IV inhibitor.

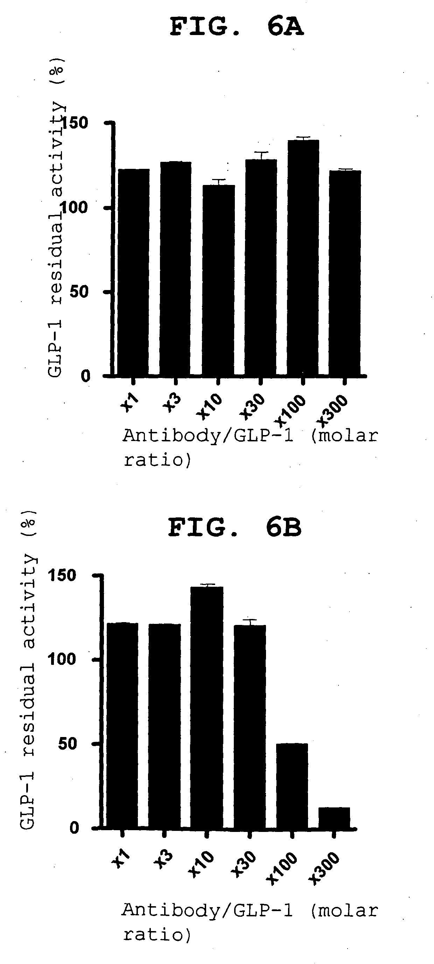

FIG. 6 shows the results of a reporter gene assay of anti-GLP-1 antibodies against GLP-1(7-36) amide [FIG. 6A: GLIT2-329(1)24 (non-neutralizing antibody) and FIG. 6B: GLIT1-492(1)2 (neutralizing antibody)]. The ordinate indicates the residual GLP-1 activity when 2 nM GLP-1(7-36)amide was reacted with each concentration of anti-GLP-1 antibody, which activity was standardized with GLP-1 activity, when reacted with the same concentration of anti-erythropoietin monoclonal antibody, as 100%. Each numerical figure on the abscissa shows the molar ratio of anti-GLP-1 antibody concentration to GLP-1(7-36) amide concentration.

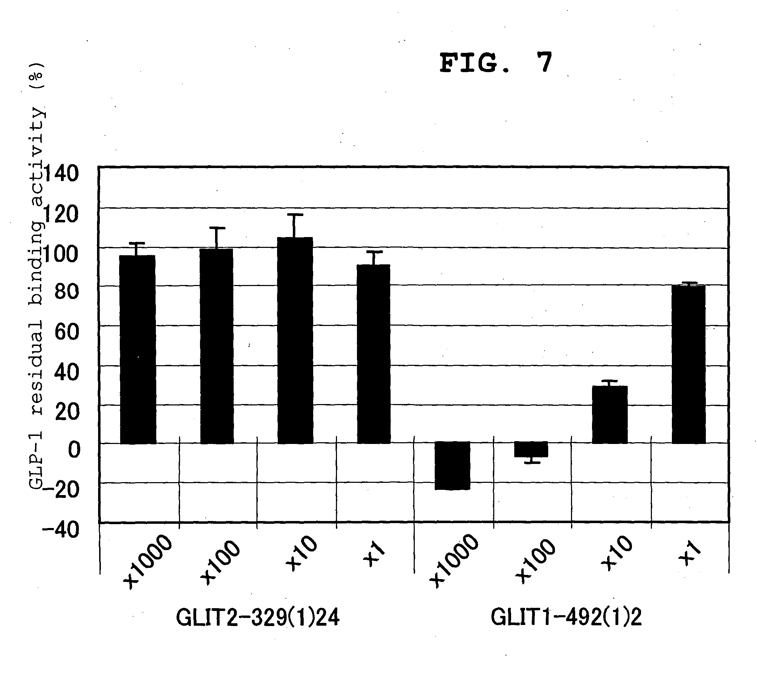

FIG. 7 shows the results of a GLP-1/GLP-1 receptor binding inhibition assay of anti-GLP-1 antibodies against GLP-1(7-36) amide [GLIT2-329(1)24 and GLIT1-492(1)2]. The ordinate indicates the ratio (residual binding activity) of $^{125}$I-labeled GLP-1 bound to GLP-1 receptor membrane fraction when 200 pM $^{125}$I-labeled GLP-1(7-36)amide was reacted with anti-GLP-1 antibody, which ratio was standardized with the amount of $^{125}$I-labeled GLP-1 bound to GLP-1 receptor membrane fraction, when reacted with the anti-PACAP38NH$_2$ non-neutralizing antibody obtained in Example 3 (PA-6Na), as 100%; each numerical figure on the abscissa shows the molar ratio of anti-GLP-1 antibody

concentration to $^{125}$I-labeled GLP-1(7-36) amide concentration.

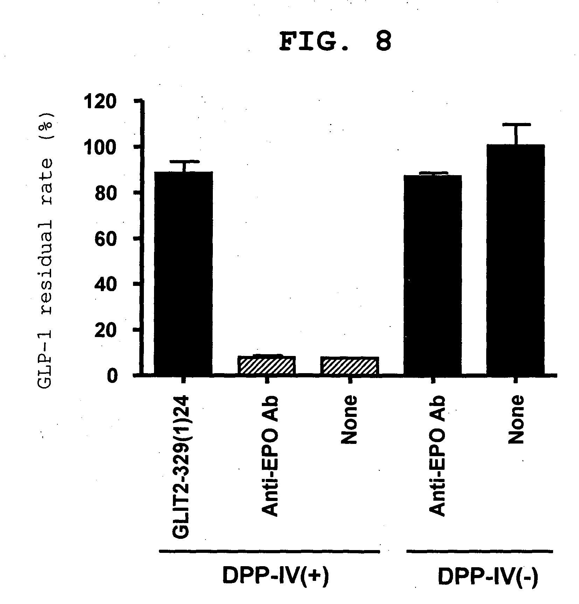

FIG. 8 shows the suppression effect of an anti-GLP-1 non-neutralizing monoclonal antibody (GLIT2-329(1)24) on the degradation of GLP-1(7-36) amide by DPP-IV. The ordinate indicates the percentage of the amount of residual GLP-1(7-36) amide after DPP-IV digestion, measured by ELISA, which was standardized with the amount of GLP-1(7-36) amide in the absence of the antibody and DPP-IV as 100%. Respective conditions are shown: GLIT2-329 (1)24: in the presence of GLIT2-329(1)24; Anti-EPO Ab: in the presence of anti-erythropoietin antibody; None: in the absence of antibody; DPP-IV(+): in the presence of DPP-IV; DPP-IV(-): in the absence of DPP-IV.

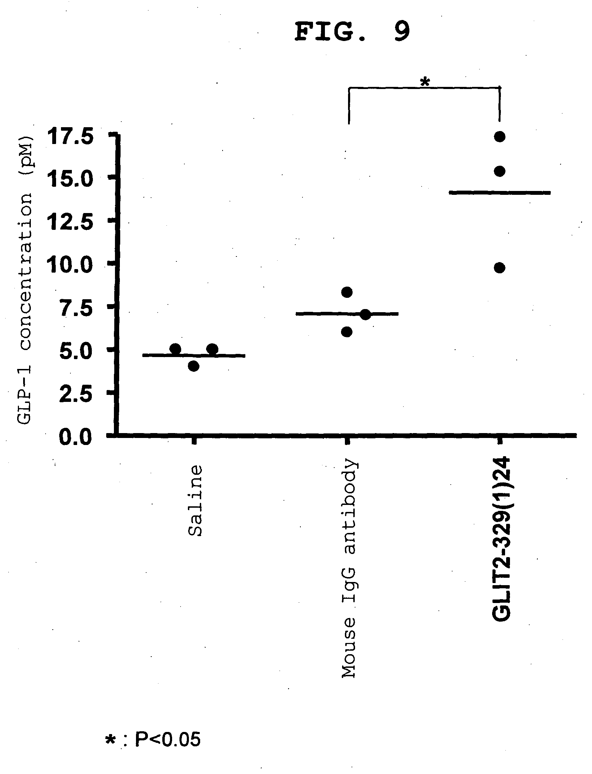

FIG. 9 shows plasma GLP-1(7-36) amide concentrations obtained one day after intraperitoneal administration of each of saline, mouse IgG (20 mg/kg) and the anti-GLP-1 non-neutralizing monoclonal antibody GLIT2-329(1)24 (20 mg/kg) to satiated Wister Fatty rats (3 animals per group).

**Best Mode for Embodying the Invention**

The antibody used in the agent for improving the blood stability of the present invention is not subject to limitation, as long as it has affinity to an endogenous ligand of a mammal (for example, human, monkey, bovine, horse, swine, sheep, goat, dog, cat, rabbit, rat, mouse, hamster, guinea pig and the like) and substantially does not neutralize the same.

As used herein, "endogenous" means naturally existing (=inherent) in a living organism (in the present invention, in the body of the mammal as the recipient of the agent for improving the blood stability of the present invention). Therefore, one administered to the animal from outside the body thereof is not included even when it is the same substance as a substance naturally occurring in a living organism.

"A ligand" means a molecule that is capable of specifically binding to a molecule that is present in cells and has the function of recognizing and transmitting external stimuli (i.e., receptor) to activate (=agonistic) or not to activate (antagonistic) the function of the receptor; examples thereof include, but are not limited to, hormones, cytokines, chemokines, growth factors, hematopoietic factors, neurotransmitters and the like.

"Substantially does not neutralize" an endogenous ligand means that the biological activity (i.e., receptor activity-regulatory action) of an endogenous ligand molecule is not inhibited at all, or an endogenous ligand molecule is inhibited only to the extent that the endogenous ligand as a whole can exhibit the biological activity necessary for the living organism. That is, there are some cases in which the desired ligand activity can be exhibited as a whole even if the activity of each endogenous ligand molecule is partially inhibited, because the antibody stabilizes the endogenous ligand to increase the blood ligand concentration, so that the antibody apparently does not neutralize the endogenous ligand. Herein, an antibody that substantially does not neutralize an endogenous ligand is hereinafter sometimes referred to as "a non-neutralizing antibody".

Preferably, the non-neutralizing antibody used in the present invention has a neutralizing activity of about 80% or less, more preferably about 50% or less, particularly preferably about 20% or less. As used herein, "neutralizing activity" means the percent inhibition of the activity of the endogenous ligand molecule. Ligand activity means receptor activity-regulatory action, and it can be measured with the activity of the receptor (effector activity-regulatory action) as the index for an agonistic ligand, or with the ability to bind to the receptor or the like as the index for an antagonistic ligand. Neutralizing activity can be calculated using the equation below.

$$\text{neutralizing activity(\%)} = ([A_{free}] - [A_{bound}]) / [A_{free}] \times 100$$

$A_{free}$: ligand activity in the absence of antibody
$A_{bound}$: ligand activity in the presence of antibody

The endogenous ligand targeted by the agent for improving the blood stability of the present invention is not subject to particular limitation, as long as the ligand's biological activity enhanced as a result of its stabilization by an antibody has an effect desirable for the living organism, and it may be any ligand molecule that has a shorter blood half-life than that of the antibody.

Preferably, the endogenous ligand that can be stabilized by the present invention is exemplified by a peptidic compound. As used herein, "a peptidic compound" means an optionally chosen compound having 1 or 2 or more peptide bonds in the molecule thereof; preferably, "a peptide" or "a protein" resulting from the polymerization of a plurality of amino acids via peptide bonds can be mentioned.

In the present description, a peptide or protein shown by an amino acid sequence is denoted with the N-terminal (amino terminal) described as the left end and the C-terminal (carboxyl terminal) as the right end, in accordance with the common way of describing peptides. The peptide or protein that is an endogenous ligand in the present invention may have any of a carboxyl group, a carboxylate, an amide or an ester at the C-terminal thereof. When the peptide or protein has a carboxyl group (or carboxylate) at a position other than the C-terminal, the carboxyl group may be amidated or esterified. Furthermore, the peptide and protein may also include a peptide or protein wherein the amino group of the N-terminal amino acid residue is substituted by formyl group, acetyl group and the like, a peptide or protein wherein the N-terminal glutamine residue has been converted to pyroglutamic acid, or a peptide or protein wherein a substituent (for example, -OH, -SH, amino group, imidazole group, indole group, guanidino group and the like) on a side chain of an amino acid in the molecule is substituted by other substituent (for example, formyl group, acetyl group and the like).

Alternatively, complex proteins (peptides) wherein a sugar chain, a fatty acid, a lipid, a nucleic acid and the like are bound to a peptide chain are also encompassed in the scope of peptides or proteins in the present invention.

As specific examples of preferable peptidic compounds as endogenous ligands, peptides or proteins that function as biologically active substances such as hormones, cytokines, chemokines, growth factors, hematopoietic factors, and neurotransmitters can be mentioned.

As examples of hormone, glucagon-like peptide-1 (GLP-1), calcitonin, pituitary adenylate cyclase-activating polypeptide (PACAP), vasoactive intestinal polypeptide (VIP), atrial natriuretic peptide (ANP), brain natriuretic peptide (BNP), C-type natriuretic peptide (CNP), leuteinizing hormone-releasing hormone (LH-RH), melanocyte-stimulating hormone (MSH), ghrelin, erythropoietin (EPO), thrombopoietin (TPO), insulin, growth hormone, leptin, adiponectin, adrenomedullin, tissue plasminogen activator (tPA), single-chain urokinase-type plasminogen activator (scu-PA), two-chain urokinase-type plasminogen activator (tcu-PA), angiotensin I, angiotensin III, angiotensin II inhibitor, bradykinin, corticotropin, dynorphin, kyotorphin, endorphin, enkephalin, secretin, growth hormone-releasing factor (GRF), neurotensin, parathyroid hormone (PTH), oxytocin, vasopressin, vasotocin, somatostatin, thyrotropin-releasing hormone (TRH), thyroid-stimulating hormone, prolactin and the like can be mentioned.

As examples of hormone cytokine, interleukins (e.g., IL-1 to IL-20), interferons (e.g., IFN$\alpha$, IFN$\beta$, IFN$\gamma$), granulocyte-macrophage colony-stimulating factor (GM-CSF), granulocyte colony-stimulating factor (G-CSF), macrophage colony-stimulating factor (M-CSF), tumor necrosis factor (TNF), lymphotoxin, T-cell replacing factor (TRF), antigen-specific suppressor factor (TsF), soluble immune response suppressor factor (SIRF), suppressor-inducing factor (SIF), macrophage activating factor (MAF), macrophage migration inhibitory factor (MIF), leukocyte migration inhibitory factor (LIF) and the like can be mentioned.

As examples of chemokine, CXC chemokines such as ENA-8, GCP-2, GRO-$\alpha$, GRO-$\beta$, GRO-$\gamma$, BCA-1, IP-10, MIG, PF-4 etc., CC chemokines such as MIP-3$\alpha$, MIP-3$\beta$, eotaxin, eotaxin-2, MCP-1 to 4, MIP-1$\alpha$, MIP-1$\beta$, RANTES etc., C chemokines such as lymphotactin etc., CX, C chemokines such as fractalkin, neurotactin etc. and the like can be mentioned.

As examples of growth factor, EGF family such as epidermal growth factor (EGF), betacellulin, betacellulin-$\delta$4, transforming growth factor-$\alpha$, (TGF-$\alpha$), heparin-binding EGF-like growth factor (HB-EGF) etc., PDGF family such as platelet-derived growth factor (PDGF), vascular endothelial growth factor (VEGF) etc., FGF family such as fibroblast growth factor (e.g., FGF-1 to -9) etc., IGF family such as insulin-like growth factor (e.g., IGF-I, IGF-II) etc., HGF family such as hepatocyte growth factor (HGF) etc., TGF-$\beta$ family such as transforming growth factor-$\beta$ (e.g., TGF-$\beta$1 to -$\beta$5) etc., NGF family such as neurotrophins (e.g., NT-1 to -5) etc., and the like can be mentioned.

As examples of hematopoietic factor, the above-mentioned EPO, TPO, GM-CSF, G-CSF, M-CSF and the like can be mentioned.

As examples of neurotransmitter, the above-mentioned dynorphin, kyotorphin, endorphin, enkephalin and the like can be mentioned.

In a preferred mode of embodiment, the agent for improving the blood stability of the present invention stabilizes a peptidic compound that is an endogenous ligand for a G protein-coupled receptor (GPCR). Examples of the GPCR include, but are not limited to, class A GPCRs such as neuropeptide receptors and chemokine receptors, class B GPCRs such as glucagon receptor, calcitonin receptor, and PTH receptor, and the like. As examples of endogenous ligands for class B GPCRs, peptides or proteins belonging to the secretin/glucagon super family, such as GLP-1, calcitonin, PACAP, and VIP, can be mentioned. As examples of endogenous ligands for GPCRs other than those of class B, LH-RH, metastin, GPR7-specific ligand [also referred to as neuropeptide B (NPB)] and ligand for both GPR7 and GPR8 [also referred to as neuropeptide W (NPW)] (these are herein generically referred to as GPR7/GPR8 ligands), MSH, ghrelin, APJ receptor-specific ligand (referred to as apelin) and the like can be mentioned.

In another preferred mode of embodiment, the agent for improving the blood stability of the present invention stabilizes a peptide or protein that is an endogenous ligand for receptors except GPCR. As examples of such peptides, ANP, BNP, CNP, beta cellulin, beta cellulin $\delta$4, adrenomedullin and the like can be mentioned; as examples of such proteins, EPO, TPO, insulin, interferons, growth hormones, GM-CSF, leptin, adiponectin and the like can be mentioned.

The above-described peptide or protein may have an amino acid sequence differing from a known amino acid sequence with respect to 1 or 2 or more amino acids, as long as it is endogenous to the recipient mammal and retains the action as a ligand. As used herein, "retains the action as a ligand" means to retain the capability of binding to the receptor, and the degree of agonism/antagonism may differ. Such examples include, but are not limited to, genetic polymorphisms, splicing variants, fragments resulting from post-translational processing, and the like. These are herein generically referred to as "analogs".

The present invention can be applied to any endogenous ligand other than peptidic compounds, as long as it can produce a non-neutralizing antibody. As such endogenous ligand, for example, ligands to GPCR or nuclear receptor such as non-peptidic hormones including steroid (e.g., glucocorticoid, estragiol, estriol, testosterone, etc.) etc., biological amine (e.g., adrenaline, dopamine, histamine, acetylcholine, noradrenaline, etc.), lipid (e.g., anandamide, cannabinoide, leukotriene, lysophosphatidic acid, platelet-activating factor, etc.), fatty acids (e.g., GPR40 ligand, etc.), eicosanoid (e.g., prostaglandin, thromboxane, etc.), bile acid (e.g., TGR5 ligand, etc.), amino acid or derivative thereof (e.g., metabolic glutamate, GABA, etc.), purine or nucleic acid (e.g., adenosine, cAMP, ATP, UTP, ADP, UDP, etc.) and the like can be mentioned.

The agent for improving the blood stability of the present invention is characterized by extending the blood half-life of an endogenous ligand by forming an immune complex with an antibody. For example, the blood half-life of a peptidic endogenous ligand like a biologically active peptide is very short because it is likely to undergo degradation by a peptidase in a living organism, because its renal clearance is fast, and because of other reasons. On the other hand, the blood half-life of an antibody is very long; for example, in the case of a whole antibody molecule, the blood half-life thereof is reportedly normally about 3 weeks. As a biologically active peptide has formed an immune complex with an antibody against the same, factors that destabilize the biologically active peptide, such as peptidases, are prevented by the antibody molecule from approaching to the target cleavage site of the peptide molecule, and the molecular size as the peptide-antibody complex increases so that the peptide is more unlikely to undergo renal clearance; for these and other reasons, the blood stability of the peptide improves [for example, peptides such as GLP-1 and PACAP are cleaved at the N-terminus thereof by dipeptidyl peptidase-IV (herein sometimes abbreviated as DPP-IV); because the resulting fragments have antagonist activity for the receptors, the agent for improving the blood stability of the present invention, which comprises a non-neutralizing antibody against these peptides, is particularly useful as a suppressor of the degradation of the peptides by DPP-IV].

Therefore, the agent for improving the blood stability of the present invention can be generally used to improve the blood stability of an endogenous ligand whose blood half-life in the free form is shorter than that of an antibody (to be specific, an endogenous ligand whose blood half-life in the free form is about 1 week or less); preferably, the agent for improving the blood stability of the present invention can be applied to an endogenous ligand whose blood half-life in the free form is about 1 day or less, more preferably about 12 hours or less, still more preferably about 6 hours or less, particularly preferably 2 hours or less, most preferably 1 hours or less and the like.

Although the antibody used in the present invention may be a monoclonal antibody or a polyclonal antibody, as long as it substantially does not neutralize the endogenous ligand that is an antigen, it is preferable to use a monoclonal antibody for obtaining a non-neutralizing antibody relatively easily because the region where antibody can bind without neutralizing the antigen is normally limited. Although the isotype of the antibody is not subject to limitation, it is preferably IgG, IgM or IgA, particularly preferably IgG.

The antibody used in the present invention is not subject to limitation, as long as it has at least a complementality determining region (CDR) for specifically recognizing and binding to the target antigen; in addition to the whole antibody molecule, the antibody may, for example, be a fragment such as Fab, Fab', or $F(ab')_2$, a genetically engineered conjugate molecule such as scFv, scFv-Fc, minibody, or diabody, or a derivative thereof modified with a molecule having protein stabilizing action, such as polyethylene glycol (PEG), or the like, and the like.

When the antibody used in the present invention is a monoclonal antibody, it can be prepared by, for example, the following method.

(1) Preparation of immunogen

As the immunogen, a compound having one or 2 or more kinds of the same epitope as the target antigen or derivative thereof can be used. For example, when the target antigen is a peptidic compound such as a peptide or protein, the antigen or derivative thereof can be obtained by (a) isolating and purifying from an antigen-producing tissue or cells of a mammal, for example, a human, monkey, rat, mouse and the like, by using a method known per se or a method based thereon, (b) chemically synthesizing by a method of peptide synthesis known per se, using a peptide synthesizer and the like (c) culturing a transformant comprising a DNA that encodes the antigen or derivative thereof, or by (d) biochemically synthesizing by using a cell-free transcription/translation system with a nucleic acid that encodes the antigen or derivative thereof as a template.

(a) When the antigen is prepared from a mammalian tissue or cells, it is possible to isolate and purify the antigen by homogenizing the tissue or cells, thereafter performing extraction with an acid or alcohol and the like, and subjecting the extract to a protein separation technique known *per se* (e.g., salting out, dialysis, chromatographies such as gel filtration chromatography, reversed phase chromatography, ionexchange chromatography, and affinity chromatography, and the like). The antigen peptide (protein) obtained can be used as the immunogen as is, and can also be used as the immunogen in the form of a partial peptide prepared by limited degradation using a peptidase and the like.

(b) When the antigen or a fragment or derivative thereof is chemically synthesized, the synthetic peptide is exemplified by ones having the same structure as the above-described antigen peptide (protein) purified from naturally occurring substances; to be specific, a peptide comprising 1 or 2 or more kinds of the same amino acid sequence as the amino acid sequence at an optionally chosen portion comprising 3 or more, preferably 6 or more, amino acids in the amino acid sequence of the antigen peptide (protein) and the like are used.

(c) When the antigen or a fragment or derivative thereof is produced using a transformant comprising a DNA, the DNA can be prepared according to a known cloning method [for example, the method described in Molecular Cloning (2nd ed.; J. Sambrook et al., Cold Spring Harbor Lab. Press, 1989) and the like]. As the cloning method, for example, a method for (i) isolating a DNA that encodes the antigen from a cDNA library by the hybridization method using DNA probes designed on the basis of the gene sequence that encodes the antigen peptide (protein), (ii) preparing a DNA that encodes the antigen or a fragment thereof by a PCR method using DNA primers designed on the basis of the gene sequence that encodes the antigen peptide (protein) with a cDNA as the template, and inserting the DNA into an expression vector matching a host, thereafter transforming the host with the expression vector, and culturing the thus-obtained transformant in a suitable medium, and the like can be mentioned.

(d) When a cell-free transcription/translation system is utilized, a method for synthesizing an mRNA by using an expression vector incorporating a DNA that encodes the antigen or a fragment thereof (for example, an expression vector wherein the DNA is placed under the control of the T7 or SP6 promoter and the like, and the like) as the template, that was prepared by the same method as (c) above, a transcription reaction mixture comprising an RNA polymerase matching the promoter, and its substrates (NTPs); and thereafter performing a translation reaction with the mRNA as the template using a known cell-free translation system (e.g., *E. coli*, rabbit reticulocytes, extract from wheat germ etc.), and the like can be mentioned. By adjusting the salt concentration and the like appropriately, the transcription reaction and the translation reaction can also be carried out in the same reaction mixture at one time.

As the immunogen, a whole antigen peptide (protein) molecule or a peptide having a partial amino acid sequence can be used. When a whole antigen peptide is used as the immunogen, selection is performed with neutralization activity and antigenic determinant mapping as the indexes. On the other hand, when antibodies recognizing a particular epitope are systemically generated and selected with a neutralizing activity as the index, a peptide having a partial amino acid sequence of an antigen peptide (protein) is used as the immunogen.

As examples of the partial amino acid sequence, those comprising 3 or more continuous amino acid residues, preferably those comprising 4 or more, more preferably 5 or more, still more preferably 6 or more continuous amino acid residues, can be mentioned. Alternatively, as examples of the amino acid sequence, those comprising 20 or less continuous amino acid residues, preferably those comprising 18 or less, more preferably 15 or less, still more preferably 12 or less continuous amino acid residues, can be mentioned. A portion of these amino acid residues (e.g., 1 to several residues) may be substituted with a substituent group (e.g., Cys, hydroxyl group, etc.). The peptide used as the immunogen has an amino acid sequence comprising one to several such partial amino acid sequences.

Such a peptide can be produced in accordance with the methods (b) to (d) above, or by cleaving an antigen peptide (protein) or a derivative thereof, prepared by the methods (a) to (d) above, with a suitable peptidase and the like; however, to prepare peptides having various partial amino acid sequences systematically, it is preferable to use a known method of peptide synthesis.

The method of peptide synthesis may, for example, be any of solid phase synthesis and liquid phase synthesis. The desired peptide can be produced by condensing a partial peptide or amino acids that can constitute the peptide and the remaining portion, and removing the protecting group when the product has a protecting group. Condensation and removal of protecting group can be achieved by known methods, for example, the methods described in (1) or (2) below.

(1) M. Bodanszky and M.A. Ondetti: Peptide Synthesis, Interscience Publishers, New York (1966)
(2) Schroeder and Luebke: The Peptide, Academic Press, New York (1965)

And after the reaction, the peptide can be purified and isolated using conventional methods of purification, such as solvent extraction, distillation, column chromatography, liquid chromatography, recrystallization, etc., in combination thereof. When the peptide obtained by the above-mentioned method is in a free form, it can be converted to a suitable salt by a known method; conversely, when the peptide is obtained in the form of a salt, the salt can be converted to a free form or other salt by a known method.

For an amide form of peptide, commercially available resins for protein synthesis, which are suitable for amide formation, can be used. As examples of such resins, chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2',4'-dimethoxyphenyl-hydroxymethyl)phenoxy resin, 4-(2',4'-dimethoxyphenyl-Fmoc-aminoethyl)phenoxy resin and the like can be mentioned. Using such resins, amino acids having α-amino groups and side-chain functional groups appropriately protected are condensed on the resin in accordance with the sequence of the desired peptide according to various condensation methods known per se. At the end of the reaction, the peptide is excised from the resin and the various protecting groups are removed simultaneously, thus affording the desired peptide. Alternatively, the desired peptide can also be obtained by using chlorotrityl resin, oxime resin, 4-hydroxybenzoic acid resin and the like, excising a partially protected peptide, and further removing protecting groups by a conventional method.

For the above-described condensation of protected amino acids, various activation reagents for peptide synthesis may be used, with preference given to carbodiimides. As the carbodiimides, DCC, N,N'-diisopropylcarbodiimide, N-ethyl-N'-(3-dimethylaminopropyl)carbodiimide and the like can be mentioned. For activation with these reagents, protected amino acids may be added directly to the resin along with a racemization inhibitor (for example, HOBt, HOOBt etc.), or may be added to the resin after being previously activated to the form of a symmetric acid anhydride, HOBt ester, or HOOBt ester. Solvents to be used in the activation of protected amino acids or condensation with the resin can be selected as appropriate from among the solvents known to be usable for peptide condensation reactions. For example, acid amides such as N,N-dimethylformamide, N,N-dimethylacetamide, and N-methylpyrrolidone; halogenated hydrocarbons such as methylene chloride and chloroform; alcohols such as trifluoroethanol; sulfoxides such as dimethylsulfoxide; tertiary amines such as pyridine; ethers such as dioxane and tetrahydrofuran; nitriles such as acetonitrile and propionitrile; esters such as methyl acetate and ethyl acetate; appropriate mixtures of these solvents, and the like can be used. Reaction temperature is selected as appropriate from the range known to be useful for peptide bond formation reactions, and is normally selected as appropriate from the range of about -20°C to about 50°C. The activated amino acid derivative is normally used in an excess of about 1.5 to about 4 times. If a test using the ninhydrin reaction reveals insufficient condensation, the condensation can be completed by repeating the condensation reaction without splitting off the protecting groups. If the condensation is yet insufficient even after repeating the reaction, unreacted amino acids can be acetylated with acetic anhydride or acetylimidazole so that an influence on the subsequent reactions can be avoided.

Protection and protecting groups for the functional groups that should not involve the reaction of the starting amino acid, splitting off the protecting groups, activation of the functional groups involved in the reaction, and the like can be selected as appropriate from among known groups or known means.

Examples of the protecting groups for the amino groups of the starting amino acid include Z, Boc, tertiary pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc and the like. As carboxyl-protecting group, for example, $C_{1-6}$ alkyl group, $C_{3-8}$ cycloalkyl group, $C_{7-14}$ aralkyl group, 2-adamantyl, 4-nitrobenzyl, 4-methoxybenzyl, 4-chlorobenzyl, phenacyl and benzyloxycarbonyl hydrazide, tertiary butoxycarbonyl hydrazide, trityl hydrazide and the like can be mentioned.

The hydroxyl group of serine or threonine can be protected by, for example, esterification or etherification. Examples of groups suitable for this esterification include lower ($C_{1-6}$) alkanoyl groups such as acetyl group, aroyl groups such as benzoyl group, groups derived from carbonic acid, such as benzyloxycarbonyl group and ethoxycarbonyl group, and the like. As examples of groups suitable for the etherification, benzyl group, tetrahydropyranyl group, t-butyl group and the like can be mentioned.

Examples of the protecting group for the phenolic hydroxyl group of tyrosine include Bzl, Cl-Bzl, 2-nitrobenzyl, Br-Z, tertiary butyl and the like.

Examples of the protecting group for the imidazole moiety of histidine include Tos, 4-methoxy-2,3,6-trimethylbenzenesulfonyl, DNP, Bom, Bum, Boc, Trt, Fmoc and the like.

Examples of activated carboxyl groups in the starting material include corresponding acid anhydrides, azides, activated esters (esters with alcohols (for example, pentachlorophenol, 2,4,5-trichlorophenol, 2,4-dinitrophenol, cyanomethyl alcohol, para-nitrophenol, HONB, N-hydroxysuccimide, N-hydroxyphthalimide, HOBt)]. Examples of activated amino groups in the starting material include corresponding phosphoric amides.

As examples of the method used to remove (split off) the protecting group, catalytic reduction in a hydrogen gas stream in the presence of a catalyst such as Pd-black or Pd-carbon; acid treatment with anhydrous hydrogen fluoride, methanesulfonic acid, trifluoromethanesulfonic acid or trifluoroacetic acid, or a mixed solution thereof; treatment with a base such as diisopropylethylamine, triethylamine, piperidine or piperazine; reduction with sodium in liquid ammonia, and the like can be mentioned. The reaction of splitting of the protecting group by the above-described acid treatment is normally performed at a temperature of about -20°C to 40°C; in the acid treatment, addition of a cation scavenger such as anisole, phenol, thioanisole, meta-cresol, para-cresol, dimethylsulfide, 1,4-butanedithiol or 1,2-ethanedithiol is effective. The 2,4-dinitrophenyl group used as the protecting group for the imidazole moiety of histidine is removed by thiophenol treatment; the formyl group used as the protecting group for the indole moiety of tryptophan is removed by alkali treatment with dilute sodium hydroxide solution, dilute ammonia or the like, as well as by the above-described acid treatment in the presence of 1,2-ethanedithiol, 1,4-butanedithiol or the like.

In another method of obtaining an amide of the peptide, for example, the $\alpha$-carboxyl group of the carboxy-terminal amino acid is first protected by amidation, and the peptide chain on the amino group side is then extended to a desired length; thereafter, a peptide having only the protecting group for the N-terminal $\alpha$-amino group in the peptide chain removed and a peptide having only the protecting group for the C-terminal carboxyl group removed are prepared, and the two peptides are condensed in a mixed solvent as described above. Details of the condensation reaction are the same as those described above. After the protected peptide obtained by the condensation is purified, all the protecting groups are removed by the above-described method to give the desired crude peptide. This crude peptide may be purified by various known means of purification, and the major fraction may be lyophilized to give an amide of the desired peptide.

An ester of the peptide can be obtained by, for example, condensing the $\alpha$-carboxyl group of the carboxy-terminal amino acid with a desired alcohol to prepare an amino acid ester, and then following the same procedures as those for the amide of the peptide.

For example, as mentioned in the Examples to be mentioned later, when PACAP38NH$_2$ or a partial peptide of PACAP38NH$_2$ is synthesized by the solid phase methods, using any of the insoluble resins known in the art such as chloromethyl resins, 4-methylbenzhydrylamine resins and 4-oxymethylphenylacetamidomethyl resins, protected amino acids are successively condensed to the C-terminal side of PACAP38NH$_2$ or of the partial peptide of PACAP38NH$_2$ according to method known in the art. Then, all protecting groups are removed by hydrogen fluoride treatment, followed by purification by methods known in the art such as high performance liquid chromatography, whereby the desired PACAP38NH$_2$ or partial peptide of PACAP38NH$_2$ can be obtained.

For example, the N-protected amino acids can be produced by protecting $\alpha$-amino groups with Boc groups (to be expressed as Boc-Xaa), the hydroxyl groups of serine and threonine with Bzl groups (to be expressed as Ser(Bzl)), the $\omega$-carboxylic acid groups of glutamic acid and aspartic acid with OBzl groups (to be expressed as Glu(OBzl)), the $\varepsilon$-amino group of lysine with a C1-Z group (to be expressed as Lys(Cl-Z)), the hydroxy group of tyrosine with a Br-Z group (to be expressed as Tyr(Br-Z)), the guanid group of arginine with a Tos group (to be expressed as Arg(Tos)), and the imidazole group of histidine with a Tos group (to be expressed as His(Tos)).

The antigen permit direct use for immunization in an insolubilized form, as long as it has immunogenicity; when an antigen of low molecular weight (for example, molecular weight about 3,000 or less) having only one to several antigenic determinants in the molecule thereof (for example, the above-described peptide and the like) is used, it can be used for immunization in the form of a complex bound or adsorbed to a suitable carrier because these antigens are normally hapten molecules of low immunogenicity. As the carrier, a naturally occurring or synthetic polymer can be used. As examples of the naturally occurring polymer, serum albumin of a mammal such as bovine, rabbit, or human, thyroglobulin of a mammal such as bovine or rabbit, ovalbumin of chicken, hemoglobin of a mammal such as bovine, rabbit, human, or sheep, keyhole limpet hemocyanin (KLH) and the like can be used. As examples of the synthetic polymer, various latexes of polymers or copolymers of polyamino acids, polystyrenes, polyacryls, polyvinyls, polypropylenes and the like, and the like can be mentioned. Regarding the mixing ratio of the carrier and hapten, any combination in any ratio can be bound or adsorbed, as long as an antibody against the antigen bound or adsorbed to the carrier is produced efficiently; usually, one wherein the above-described naturally occurring or synthetic polymer carrier in common use in preparing an antibody against hapten is bound or adsorbed in a ratio by weight of 0.1 to 100 to 1 of hapten can be used.

Various condensing agents can be used for coupling the hapten and carrier. For example, diazonium compounds such as bisdiazotized benzidine, which crosslink tyrosine, histidine, and tryptophan; dialdehyde compounds such as glutaraldehyde, which crosslink amino groups together; diisocyanate compounds such as toluene-2,4-diisocyanate; dimaleimide compounds such as N,N'-o-phenylenedimaleimide, which crosslink thiol groups together; maleimide activated ester compounds, which crosslink amino groups and thiol groups; carbodiimide compounds, which crosslink amino groups and carboxyl groups; and the like can be used advantageously. When amino groups are crosslinked together, it is also possible to react one amino group with an activated ester reagent having a dithiopyridyl group (for example, SPDP and the like), followed by reduction, to introduce the thiol group, and to introduce a maleimide group into the other amino group using a maleimide activated ester reagent, followed by a reaction of both.

(2) Preparation of monoclonal antibody

An antigen is administered as is, or along with a carrier or a diluent, to a warm-blooded animal at a site enabling antibody production by the methods such as intraperitoneal injection, intravenous injection, subcutaneous injection, intradermal injection and the like. In order to increase antibody productivity upon the administration, Freund's complete adjuvant or Freund's incomplete adjuvant may be administered. Dosing is normally performed about two to 10 times in total every 1 to 6 weeks. As examples of the warm-blooded animal used, monkeys, rabbits, dogs, guinea pigs, mice, rats, sheep, goats, donkeys and chickens can be mentioned. Although it is preferable to use a mammal of the same species as the recipient in order to avoid the problem of anti-Ig antibody production, mice and rats are generally preferably used for generating a monoclonal antibody.

Because artificial immunization to humans is ethically difficult, it is preferable, when the agent for improving the blood stability of the present invention targets a human, (i) to obtain a human antibody by immunizing a human antibody-producing animal (e.g., mouse) produced according to a method described below, (ii) to produce a chimeric antibody, humanized antibody or fully human antibody according to a method described below, or (iii) to obtain a human antibody using in combination the in vitro immunization method and cell immortalization with virus, human-human (or -mouse) hybridoma production technique, phage display method and the like. Note that the *in vitro* immunization method can also be used preferably as a method for obtaining an antigen against an antigen that is unstable and difficult to prepare in large amounts for the purpose of preparing a non-human animal-derived antibody, because there is the possibility of obtaining an antibody against an antigen for which antibody production is suppressed by ordinary immunization, because it is possible to obtain an antibody with an amount of antigen on the nanogram to microgram order, because immunization completes in several days, and for other reasons.

As the animal cells used in the in vitro immunization method, lymphocytes, preferably B-lymphocytes and the like, isolated from peripheral blood, spleen, lymph node and the like of a human and the above-described warm-blooded animals (preferably mouse or rat) can be mentioned. For example, in the case of mouse or rat cells, the spleen is extirpated from an about 4- to 12-week-old animal, and splenocytes are separated and rinsed with a appropriate medium [e.g., Dulbecco's modified Eagle medium (DMEM), RPMI1640 medium, Ham's F12 medium and the like], after which the splenocytes are suspended in an antigen-containing medium supplemented with fetal calf serum (FCS; about 5 to 20%) and cultured using a $CO_2$ incubator and the like for about 4 to 10 days. Examples of the antigen concentration include, but are not limited to, 0.05 to 5 μg. It is preferable to prepare a culture supernatant of thymocytes of an animal of the same strain (preferably at about 1 to 2 weeks of age) according to a conventional method, and to add the supernatant to the medium.

Because it is difficult to obtain a thymocyte culture supernatant in *in vitro* immunization of human cells, it is preferable to perform immunization by adding, to the medium, several kinds of cytokines such as IL-2, IL-4, IL-5, and IL-6 and the like, and if necessary, an adjuvant substance (e.g., muramyldipeptide and the like) along with the antigen.

In preparing a monoclonal antibody, it is possible to establish an antibody-producing hybridoma by selecting an individual or cell population showing an elevated antibody titer from among antigen-immunized warm-blooded animals (e.g., mice, rats) or animal cells (e.g., human, mouse, rat), respectively; collecting spleens or lymph nodes at 2 to 5 days after the final immunization or collecting the cells after 4 to 10 days of cultivation after *in vitro* immunization to isolate antibody-producing cells; and fusing the isolated cells with myeloma cells. A measurement of serum antibody titer can be performed by, for example, reacting a labeled antigen and an antiserum, and thereafter determining the activity of the label bound to the antibody.

Although the myeloma cells are not subject to limitation, as long as they are capable of producing a hybridoma that secretes a large amount of antibody, those that do not produce or secrete the antibody per se are preferable, with greater preference given to those of high cell fusion efficiency. To facilitate hybridoma selection, it is preferable to use a cell line that is susceptible to HAT (hypoxanthine, aminopterin, thymidine). As examples of the mouse myeloma cells, NS-1, P3U1, SP2/0, AP-1 and the like can be mentioned; as examples of the rat myeloma cells, R210.RCY3, Y3-Ag 1.2.3 and the like can be mentioned; as examples of the human myeloma cells, SKO-007, GM 1500-6TG-2, LICR-LON-HMy2, UC729-6 and the like can be mentioned.

Fusion operation can be performed according to a known method, for example, the method of Koehler and Milstein [*Nature*, *256*, 495 (1975)]. As a fusion promoter, polyethylene glycol (PEG), Sendai virus and the like can be mentioned, and PEG and the like are preferably used. Although the molecular weight of PEG is not subject to limitation, PEG1000 to PEG6000, which are of low toxicity and relatively low viscosity, are preferable. As examples of the PEG concentration, about 10 to 80%, preferably about 30 to 50%, can be mentioned. As the solution for diluting PEG, various buffers such as serum-free medium (e.g., RPMI1640), complete medium comprising about 5 to 20% serum, phosphate buffered saline (PBS), and Tris buffer can be used. DMSO (e.g., about 10 to 20%) can also be added as desired. As examples of the pH of the fusion solution, about 4 to 10, preferably about 6 to 8 can be mentioned.

The ratio by number of antibody-producing cells (splenocytes) and myeloma cells is preferably about 1:1 to 20:1, and the cell fusion can be efficiently performed by incubation normally at 20 to 40˚C, preferably at 30 to 37˚C, normally for 1 to 10 minutes.

An antibody-producing cell line can also be obtained by infecting antibody-producing cells with a virus capable of transforming lymphocytes to immortalize the cells. As such viruses, for example, Epstein-Barr (EB) virus and the like can be mentioned. Although the majority of persons have immunity because they have ever been infected with this virus in an asymptomatic infection of infectious mononucleosis, virion is also produced when the ordinary EB virus is used; therefore, appropriate purification must be performed. As an EB system free from the possibility of viral contamination, it is also preferable to use a recombinant EB virus that retains the capability of immortalizing B lymphocytes but lacks the capability of replicating virion (for example, deficiency of the switch gene for transition from latent infection state to lytic infection state and the like).

Because marmoset-derived B95-8 cells secrete EB virus, B lymphocytes can be easily transformed by using a culture supernatant thereof. An antibody-producing B cell line can be obtained by, for example, culturing these cells using a medium supplemented with serum and penicillin/streptomycin (P/S) (e.g., RPMI1640) or a serum-free medium supplemented with a cell growth factor, thereafter separating the culture supernatant by filtration or centrifugation and the like, suspending therein antibody-producing B lymphocytes at a suitable concentration (e.g., about $10^7$ cells/mL), and incubating the suspension normally at 20 to 40°C, preferably at 30 to 37°C, normally for about 0.5 to 2 hours. When human antibody-producing cells are provided as mixed lymphocytes, it is preferable to previously remove T lymphocytes by allowing them to form an E rosette with, for example, sheep erythrocytes and the like, to increase transformation frequency of EB virus, because the majority of persons have T lymphocytes which exhibit cytotoxicity to cells infected with EB virus. It is also possible to select lymphocytes specific for the target antigen by mixing sheep erythrocytes, previously bound with a soluble antigen, with antibody-producing B lymphocytes, and separating the rosette using a density gradient of percoll and the like. Furthermore, because antigen-specific B lymphocytes are capped by adding the antigen in large excess so that they no longer present IgG to the surface, mixing with sheep erythrocytes bound with anti-IgG antibody results in the formation of rosette only by antigen-nonspecific B lymphocytes. Therefore, by collecting a layer of cells that don't form rosette from this mixture using a density gradient of percoll and the like, it is possible to select antigen-specific B lymphocytes.

Human antibody-secreting cells having acquired the capability of proliferating indefinitely by the transformation can be back fused with mouse or human myeloma cells in order to stably sustain the antibody-secreting ability. As the myeloma cells, the same as those described above can be used.

Hybridoma screening and breeding are normally performed using a medium for animal cells (e.g., RPMI1640) containing 5 to 20% FCS or a serum-free medium supplemented with cell growth factors, with the addition of HAT (hypoxanthine, aminopterin, thymidine). As examples of the concentrations of hypoxanthine, aminopterin and thymidine, about 0.1 mM, about 0.4 $\mu$M and about 0.016 mM and the like, respectively, can be mentioned. For selecting a human-mouse hybridoma, ouabain resistance can be used. Because human cell lines are more susceptible to ouabain than mouse cell lines, it is possible to eliminate unfused human cells by adding ouabain at about $10^{-7}$ to $10^{-3}$ M to the medium.

In selecting a hybridoma, it is preferable to use feeder cells or culture supernatants of certain cells. As the feeder cells, an allogenic cell species having a lifetime limited so that it dies after helping the emergence of hybridoma, cells capable of producing large amounts of a growth factor useful for the emergence of hybridoma with their proliferation potency reduced by irradiation and the like, and the like are used. For example, as the mouse feeder cells, splenocytes, macrophage, blood, thymocytes and the like can be mentioned; as the human feeder cells, peripheral blood mononuclear cells and the like can be mentioned. As examples of the cell culture supernatant, primary culture supernatants of the above-described various cells and culture supernatants of various established cell lines can be mentioned.

Moreover, a hybridoma can also be selected by reacting a fluorescein-labeled antigen with fusion cells, and thereafter separating the cells that bind to the antigen using a fluorescence-activated cell sorter (FACS). In this case, efforts for cloning can be lessened significantly because a hybridoma that produces an antibody against the target antigen can be directly selected.

For cloning a hybridoma that produces a monoclonal antibody against the target antigen, various methods can be used.

It is preferable to remove aminopterin as soon as possible because it inhibits many cell functions. In the case of mice and rats, aminopterin can be removed 2 weeks after fusion and beyond because most myeloma cells die within 10 to 14 days. However, a human hybridoma is normally maintained in a medium supplemented with aminopterin for about 4 to 6 weeks after fusion. It is desirable that hypoxanthine and thymidine be removed more than one week after the removal of aminopterin. That is, in the case of mouse cells, for example, a complete medium (e.g., RPMI1640 supplemented with 10% FCS) supplemented with hypoxanthine and thymidine (HT) is added or exchanged 7 to 10 days after fusion. About 8 to 14 days after fusion, visible clones emerge. Provided that the diameter of clone has reached about 1 mm, the amount of antibody in the culture supernatant can be measured.

A measurement of the amount of antibody can be performed by, for example, a method comprising adding the hybridoma culture supernatant to a solid phase (e.g., microplate) to which the target antigen or a derivative thereof or partial peptide thereof (including the partial amino acid sequence used as the epitope) is adsorbed directly or with a carrier, subsequently adding an anti-immunoglobulin (IgG) antibody (an antibody against IgG derived from an animal of the same species as the animal from which the original antibody-producing cells are derived is used) or protein A, which had been labeled with a radioactive substance (e.g., $^{125}$I, $^{131}$I, $^3$H, $^{14}$C), enzyme (e.g., $\beta$-galactosidase, $\beta$-glucosidase, alkaline phosphatase, peroxidase, malate dehydrogenase), fluorescent substance (e.g., fluorescamine, fluorescein isothiocyanate), luminescent substance (e.g., luminol, luminol derivative, luciferin, lucigenin) and the like, and detecting the antibody against the target antigen (epitope) bound to the solid phase, a method comprising adding the hybridoma culture supernatant to a solid phase to which an anti-IgG antibody or protein A is adsorbed, adding the target antigen, a derivative thereof, or a partial peptide thereof labeled with the same labeling reagent as described above, and detecting the antibody against the target antigen (epitope) bound to the solid phase and the like.

Although limiting dilution is normally used as the cloning method, cloning using soft agar and cloning using FACS (described above) are also possible. Cloning by limiting dilution can be performed by, for example, the following procedures, which, however, are not to be construed as limiting.

The amount of antibody is measured as described above, and positive wells are selected. Selected suitable feeder cells are previously added to a well plate. Cells are collected from the antibody-positive wells and suspended in complete medium (e.g., RMPI1640 supplemented with 10% FCS and P/S) to obtain a density of 30 cells/mL; 0.1 mL (3 cells/well) of this suspension is added to the 96-well plate with feeder cells added thereto; a portion of the remaining cell suspension is diluted to 10 cells/mL and sown to other wells (1 cell/well)in the same way; the still remaining cell suspension is diluted to 3 cells/mL and sown to other wells (0.3 cells/well). The cells are cultured for about 2 to 3 weeks until a visible clone appears, when the amount of antibody is measured to select positive wells, and the selected cells are recloned in the same way. In the case of human cells, cloning is relatively difficult, so that a plate in which cells are seeded at 10 cells/well is also prepared. Although a monoclonal antibody-producing hybridoma can be obtained normally by two times of subcloning, it is desirable to repeat recloning regularly for several more months to confirm the stability thereof.

Hybridomas can be cultured *in vitro* or *in vivo.*

As a method of *in vitro* culture, a method comprising gradually scaling up a monoclonal antibody-producing hybridoma obtained as described above, from a well plate, while keeping the cell density at, for example, about $10^5$ to $10^6$ cells/mL, and gradually lowering the FCS concentration, can be mentioned.

As a method of *in vivo* culture, for example, a method comprising an intraperitoneal injection of a mineral oil to a mouse (a mouse that is histocompatible with the parent strain of the hybridoma) to induce plasmacytoma (MOPC) 5 to 10 days later, to which intraperitoneally injecting about $10^6$ to $10^7$ cells of hybridoma, and collecting ascites fluid under anesthesia 2 to 5 weeks later, can be mentioned.

Separation and purification of the monoclonal antibody are performed according to a method of immunoglobulin separation and purification [e.g., salting-out, alcohol precipitation, isoelectric point precipitation, electrophoresis, adsorption-desorption with an ion exchanger (e.g., DEAE, QEAE), ultracentrifugation, gel filtration, specific purification comprising selectively collecting the antibody by means of an antigen-coupled solid phase or an active adsorbent such as protein A or protein G, and dissociating the linkage to obtain the antibody, and the like] in the same manner as an ordinary separation and purification of the polyclonal antibody.

As described above, a monoclonal antibody can be produced by culturing a hybridoma in or outside the living body of a warm-blooded animal, and harvesting an antibody from the body fluid or culture thereof.

Because the antibody used in the present invention must be one that substantially does not neutralize the endogenous ligand as the antigen, it is necessary to examine the degree of neutralization activity of the monoclonal antibody obtained. The neutralization activity can be measured by comparing the effector activity-regulatory action of a receptor for the ligand, or ligand-receptor binding ability, between in the presence and absence of the antibody. For example, when the endogenous ligand is for a GPCR that couples with G protein, which promotes or suppresses adenylate cyclase (AC) activity, the neutralization activity can be measured by, in the presence of the ligand alone and in the presence of a ligand-antibody complex using, for example, 1) a method comprising adding ATP to cells or a membrane fraction thereof, comprising the GPCR and AC on the cell membrane thereof, and measuring the amount of resulting cAMP by a competitive immunoassay with cAMP labeled with a radioactive substance (e.g., $^{125}$I), enzyme (e.g., alkaline phosphatase, peroxidase), fluorescent substance (e.g., FITC, rhodamine) and the like, using an anti-cAMP antibody, 2) a method comprising adding $[\alpha\text{-}^{32}\text{P}]$ATP to the above-described cells or a membrane fraction thereof, separating the resulting $[^{32}\text{P}]$cAMP using an alumina column and the like, and thereafter measuring the radioactivity thereof, 3) a method comprising measuring the expression level of a reporter gene (e.g., luciferase gene) in transformant cells transfected with the gene under the control of a cAMP responsive element (CRE), 4) a method comprising adding $[^{35}\text{S}]$GTP$\gamma$S (a GTP analog not undergoing hydrolysis by the GTPase activity of G protein $\alpha$-subunit) to a membrane fraction comprising the GPCR, and measuring the radioactivity bound to the membrane, 5) a method comprising measuring the binding of the GPCR and the ligand by a competitive immunoassay with the ligand labeled with a radioactive substance (e.g., $^{125}$I), enzyme (e.g., alkaline phosphatase, peroxidase), fluorescent substance (e.g., FITC, rhodamine) and the like, and the like. When the receptor for the endogenous ligand is a GPCR that has phospholipase C (PLC) as the effector, there can be used, in place of the above-described methods 1) to 3), 1) a method comprising adding phosphatidylinositol-4,5-bisphosphate to cells or a membrane fraction thereof, comprising the GPCR and PLC in the cell membrane thereof, and measuring the amount of the resulting inositol phosphate, 2) a method comprising measuring the amount of intracellular $Ca^{2+}$ in cells comprising the GPCR and PLC in the cell membrane thereof, 3) a method comprising measuring the expression level of a reporter gene, in transformant cells transfected with the reporter gene under the control of TPA (12-0-tetradecanoylphorbol-13-acetate) responsive element (TRE), which is upregulated by $Ca^{2+}$, and the like. Note that the amount of intracellular $Ca^{2+}$ can be measured spectroscopically using a fluorescent probe (fura-2, indo-1, fluor-3, Calcium-Green I and the like) or can be measured using aequorin which is a calcium-sensitive photoprotein, and the like. As an apparatus suitable for the spectroscopic measurement using a fluorescent probe, the FLIPR (Molecular Devices Company) system can be mentioned.

As a result of performing the above-described assay, for example, an antibody having a neutralization activity of about 80% or less, preferably about 50% or less, more preferably about 20% or less, can be selected as a candidate for the non-neutralizing antibody used in the present invention.

In a preferred mode of embodiment, because the agent for improving the blood stability of the present invention is a pharmaceutical product having humans as the subject of administration thereof, the antibody used in the present invention (preferably a monoclonal antibody) is an antibody whose risk of showing antigenicity when administered to a human has been reduced; to be specific, the antibody is a fully human antibody, a humanized antibody, a mouse-human chimeric antibody and the like, particularly preferably a fully human antibody. A humanized antibody and a chimeric antibody can be prepared by genetic engineering technology according to the method described below. Although a fully human antibody can also be produced from the above-described human-human (or -mouse) hybridoma, it is desirable to produce it using a human antibody-producing animal described below (e.g., mouse) or the phage display method in order to stably supply the antibody in large amounts at low costs.

(1) Preparation of chimeric antibody

As used herein, "a chimeric antibody" means an antibody wherein the sequences of the variable regions of the H chain and L chain ($V_H$ and $V_L$) thereof are derived from a mammalian species, and wherein the sequences of the constant regions ($C_H$ and $C_L$) are derived from another mammalian species. The sequences of the variable regions are preferably derived from, for example, an animal species permitting easy preparation of a hybridoma, such as mouse, and the sequences of the constant regions are preferably derived from the recipient mammalian species.

As examples of the method of preparing a chimeric antibody, the method described in US Patent No. 6,331,415 or a partially modified method thereof and the like can be mentioned. To be specific, first, mRNA or total RNA is prepared from a monoclonal antibody-producing hybridoma (for example, mouse-mouse hybridoma) obtained as described above, according to a conventional method, to synthesize cDNA. DNAs that encode $V_H$ and $V_L$ are amplified and purified by PCR according to a conventional method with the cDNA as the template, using appropriate primers [for example, oligo DNAs comprising the base sequences that encode the N-terminal sequences of $V_H$ and $V_L$, respectively, as the sense primers, and oligo DNAs that hybridize to the base sequences that encode the terminal sequences of $C_H$ and $C_L$, respectively, as the antisense primer (see, for example, Bio/Technology, 9: 88-89, 1991)]. In the same manner, DNAs that encode $C_H$ and $C_L$ are amplified and purified from an RNA prepared from lymphocytes and the like of another mammal (e.g. , human) by RT-PCR. $V_H$ and $C_H$, and $V_L$ and $C_L$, are ligated together, respectively, using a conventional method, and the chimeric H chain DNA and chimeric L chain DNA obtained are inserted into respective appropriate expression vectors [for example, vectors comprising promoters that have transcription activity in CHO cells, COS cells, mouse myeloma cells and the like (e.g., CMV promoter, SV40 promoter and the like)]. The DNAs that encode the two chains may be inserted into separate vectors, and may be inserted into a single vector in tandem. Host cells are transformed with the chimeric H chain and chimeric L chain expression vector(s) obtained. As the host cells, animal cells, for example, Chinese hamster ovary (CHO) cells, monkey-derived COS-7 cells, Vero cells, rat-derived GHS cells and the like, in addition to the above-described mouse myeloma cells, can be mentioned. For the transformation, any method applicable to animal cells can be used, with preference given to electroporation method and the like. It is possible to isolate a chimeric monoclonal antibody by culturing the host cells in a medium suitable thereto for a given period, and thereafter recovering the culture supernatant and purifying it in the same manner as described above. Alternatively, it is also possible to obtain a chimeric monoclonal antibody easily and in large amounts from milk or eggs of transgenic animals which are produced by a conventional method using germ line cells of an animal such as bovine, goat, or chicken as the host cells, for which a transgenic technique has been established and a know-how of mass propagation as a domestic animal (domestic fowl) has been compiled. Furthermore, it is also possible to obtain a chimeric monoclonal antibody in large amounts from the seeds, leaves and the like of a transgenic plant, produced by using microinjection and electroporation into protoplast, the particle gun method and Ti-vector method for intact cells and the like, with cells of a plant such as corn, rice, wheat, soybean, or tobacco as the host cells, for which a transgenic technique has been established, and which is cultured in large amounts as a major crop.

When the chimeric monoclonal antibody obtained is digested with papain, Fab is obtained; when the same is digested with pepsin, $F(ab')_2$ is obtained.

It is also possible to reformat into scFv by ligating DNAs that encode mouse $V_H$ and $V_L$ via a suitable linker, for example, a DNA that encodes a peptide consisting of 1 to 40 amino acids, preferably 3 to 30 amino acids, more preferably 5 to 20 amino acids [e.g., [Ser-(Gly)m]n or [(Gly)m-Ser]n (m is an integer from 0 to 10, n is an integer from 1 to 5) and the like]. Furthermore, it is possible to reformat into a minibody by ligating a DNA that encodes $C_{H3}$ via a suitable linker thereto, or reformat into a scFv-Fc by ligating a DNA that encodes $C_H$ full length via a suitable linker thereto. The DNA encoding such an antibody molecule modified (coupled) by genetic engineering can be expressed in a microorganism such as *E. coli* or yeast under the control of a suitable promoter, to produce the antibody molecule in large amounts.

When DNAs encoding mouse $V_H$ and $V_L$ are inserted into the downstream of one promoter in tandem and introduced into E. coli, a dimer named as Fv is formed by monocistronic gene expression. When an appropriate amino acid in the FRs of $V_H$ and $V_L$ is substituted with Cys using molecule modeling, a dimer named as dsFv is formed via the intermolecular disulfide bond between the two chains.

(2) Humanized antibody

As used herein, "a humanized antibody" means an antibody wherein the sequences of all regions present in the variable region, other than the complementality determining region (CDR), [i.e., framework region (FR) in constant region and variable region] are derived from a human, and wherein only the sequence of CDR is derived from another mammalian species. The other mammalian species is preferably an animal species, for example, mouse and the like, with which production of hybridomas can be easily performed.

As examples of the method of preparing a humanized antibody, the methods described in US Patent Nos. 5,225,539, 5,585,089, 5,693,761 and 5,693,762 or partially modified methods therefrom and the like can be mentioned. To be specific, DNAs that encode $V_H$ and $V_L$ derived from a non-human mammalian species (e.g., mouse) are isolated in the same manner as with the above-described chimeric antibody, after which sequencing is performed by a conventional method using an automated DNA sequencer (e.g., manufactured by Applied Biosystems Company and the like), and the base sequences obtained or deduced amino acid sequences therefrom are analyzed using a known antibody sequence database [for example, Kabat database (see Kabat et al., "Sequences of Proteins of Immunological Interest", edited by NIH, US Department of Health and Human Services, Public Health Service, 5th edition, 1991) and the like] to determine the CDR and FR of the two chains. A base sequence wherein the CDR encoding region of a base sequence that encodes the L chain and H chain of a human antibody having an FR sequence similar to the determined FR sequence [e.g., human κ type L chain subgroup I and human H chain subgroup II or III (see Kabat et al., 1991 (*supra*))] is substituted with the determined base sequence that encodes the CDR of another animal species, is designed, and the base sequence is divided into fragments of about 20 to 40 bases, and a sequence complementary to the base sequence is divided into fragments of about 20 to 40 bases so that they alternatively overlap with the aforementioned fragments. It is possible to construct DNAs that encode $V_H$ and $V_L$ having human-derived FR and a CDR derived from another mammalian species by synthesizing individual fragments using a DNA synthesizer, and hybridizing and ligating them in accordance with conventional methods. In order to transfer a CDR derived from another mammalian species into human-derived $V_H$ and $V_L$ more quickly and more efficiently, it is preferable to use PCR-based site directed mutagenesis. As examples of such a method, the sequential CDR grafting method described in Japanese Patent Unexamined Publication No. HEI-5-227970 and the like can be mentioned. It is possible to obtain cells or transgenic animal/plant that produces a humanized antibody by ligating the thus-obtained DNAs that encode $V_H$ and $V_L$ to DNAs that encode human-derived $C_H$ and $C_L$, respectively, in the same manner as with the above-described chimeric antibody, and introducing the ligated product into suitable host cells.

A humanized antibody, like a chimeric antibody, can be modified to scFv, stFv-Fc, minibody, dsFv, Fv and the like by using genetic engineering techniques; and they can be produced in a microorganism such as E. coli or yeast by using a suitable promoter.

The technology for preparing a humanized antibody can also be applied to, for example, preparing a monoclonal antibody that can be preferably administered to another animal species for which no hybridoma production technology has been established. For example, animals widely propagated as domestic animals (domestic fowls) such as bovine, swine, sheep, goat, and chicken, and pet animals such as dogs and cats, and the like can be mentioned as the subject animal species.

(3) Preparation of fully human antibody using human antibody-producing animal

Provided that a functional human Ig gene is introduced into a non-human warm-blooded animal having the endogenous immunoglobulin (Ig) gene knocked out (KO) therein, and that this animal is immunized with an antigen, a human antibody is produced in place of the antibody derived from the animal. Therefore, provided that an animal such as mice, for which a technique for producing a hybridoma has been established, is used, it is possible to acquire a fully human monoclonal antibody by the same method as the conventional method used to prepare a mouse monoclonal antibody. First, some of the human monoclonal antibodies, that were generated by using a human antibody-producing mouse obtained by crossing a mouse transfected with minigenes of the human Ig H chain and L chain using an ordinary transgenic (Tg) technique with a mouse wherein the endogenous mouse Ig gene has been inactivated using an ordinary KO technique, are already in clinical stage, and to date production of anti-human Ig human antibody (HAHA) has not been reported.

Later, Abgenix Inc. [trade name: XenoMouse (see Nat. Genet., 15: 146-156, 1997; US Patent No. 5,939,598 and the like)] and Medarex Inc. [trade name: Hu-Mab Mouse (see Nat. Biotechnol., 14: 845-851, 1996; US Patent No. 5,545,806 and the like)] established Tg mice transfected with even a larger human Ig gene using a yeast artificial chromosome (YAC) vector, thus enabling the production of human antibodies of richer repertoire. However, because the human Ig gene, for example, in the case of the H chain, exhibits its diversity as the VDJ exon, which is a variable combination of about 80 kinds of V fragments, about 30 kinds of D fragments and 6 kinds of J fragments, encodes the antigen binding site, the full length thereof is as large as about 1.5 Mb (14th chromosome) for the H chain, about 2 Mb (2nd chromosome) for the $_\kappa$L chain, and about 1 Mb (22nd chromosome) for the $\lambda$L chain. To reproduce the diverse antibody repertoire in human in another animal species, it is desirable to introduce the full length of each Ig gene. However, a DNA that is insertable into a conventional transfection vector (plasmid, cosmid, BAC, YAC and the like) is normally several kb to several hundred kb in length, and it has been difficult to introduce the full length of Ig genes by the conventional technique for establishing a transgenic animal, which comprises inserting a cloned DNA into a fertilized egg.

Tomizuka et al. *(Nat. Genet., 16*: 133-143, 1997) prepared a mouse having the full-length human Ig gene by introducing a natural fragment of a human chromosome harboring the Ig gene (hCF) into a mouse [transchromosomic (TC) mouse]. That is, first, a human-mouse hybrid cell having human chromosomes in which the 14th chromosome comprising the H chain gene and the 2nd chromosome comprising the $_\kappa$L chain gene, both labeled with, for example, a drug-resistance marker and the like, is treated with a spindle formation inhibitor (e.g., colcemid) for about 48 hours to prepare a microcell wherein one to several chromosomes or fragments thereof are enveloped in nuclear membrane, and the chromosomes are introduced into a mouse ES cell by the micronuclear fusion method. A hybrid ES cell retaining the chromosomes having the human Ig gene or fragments thereof is selected using a medium containing a drug, and the cell is microinjected into a mouse embryo in the same manner as with the preparation of an ordinary KO mouse. A germ line chimera is selected among the chimeric mice obtained, with coat color as the index, and the like, to establish a TC mouse strain carrying the human 14th chromosome fragment (TC(hCF14)) and a TC mouse strain carrying the human 2nd chromosome fragment (TC(hCF2)). After establishing mouse strains wherein the endogenous H chain gene and $\kappa$L chain gene are knocked out, respectively [KO (IgH) and KO (Ig$\kappa$)] by a conventional method, it is possible to establish a mouse strain having all the four kinds of gene modifications (double TC/KO) by repeating the crossing of these four strains.

Provided that the same method as that for producing an ordinary mouse monoclonal antibody is applied to a double TC/KO mouse established as described above, it is possible to obtain an antigen-specific human monoclonal antibody-producing hybridoma. However, there is the drawback of a lower efficiency to obtain hybridomas than that with the ordinary mouse, because hCF2 containing the $_\kappa$L chain gene is unstable in the mouse cells.

On the other hand, because the aforementioned Hu-Mab mouse has a structure wherein the variable region cluster are doubled although it has about 50% of the $_\kappa$L chain gene, it exhibits a $\kappa$ chain diversity equivalent to that with full length (on the other hand, HuMab mouse exhibits a low H chain diversity and inadequate response to antigen because it carries only about 10% of the H chain gene). And the $\kappa$ chain is stably retained in the mouse cells because it is inserted in mouse chromosome via a YAC vector (Ig$_\kappa$-YAC). Making use of this advantage, it is possible to get the efficiency for obtaining hybridomas and affinity to antigen affinity of antibody that are equivalent to those with the ordinary mouse, by crossing a TC(hCF14) mouse with a Hu-Mab mouse to establish a mouse that stably retains both hCF14 and Ig$_\kappa$-YAC (trade name: KM mouse).

Furthermore, it is also possible to establish a human antibody-producing animal in which the $\lambda$L chain gene is further transfected to reconstruct the diverse human antibody repertoire more completely. Such an animal can also be obtained by producing a TC mouse in which the human 22nd chromosome or a fragment thereof harboring the $\lambda$L chain gene is introduced in the same manner as described above [TC(hCF22)], and crossing the mouse with the above-described double TC/KO mouse or KM mouse, or can also be obtained by, for example, constructing a human artificial chromosome (HAC) comprising both the H chain locus and the $\lambda$L chain locus, and introducing it into a mouse cell *(*Nat. Biotechnol., 18: 1086-1090, 2000).

An antibody used in the present invention is desirably a monoclonal antibody because it must be a non-neutralizing antibody. However, it is possible in principle to obtain a non-neutralizing antibody even if it is a polyclonal antibody because an antibody that binds to the specific site of the target antigen can be obtained using a small hapten molecule as the antigen. When the antibody used in the present invention is a polyclonal antibody, it is not necessary to use hybridomas; therefore, provided that a human antibody-producing animal is produced in the same manner as described above using an animal species for which no technique for preparing a hybridoma has been established but a transgenic technique has been established, preferably an ungulate such as bovine, it is also possible to produce a human antibody in larger amounts at low costs (see, for example, Nat. Biotechnol., 20: 889-894, 2002). The human polyclonal antibody thus obtained can be purified by collecting blood, ascites fluid, milk, egg and the like, preferably milk or egg, of the human antibody-producing animal, in combination with the same purification techniques as described above.

(4) Preparation of fully human antibody using phage display human antibody library

Another approach to produce a fully human antibody is a method using phage display. This method sometimes encounters cases in which a mutation due to PCR is introduced into a site other than CDRs; for this reason, a few reports of cases of HAHA production in clinical stage are available. On the other hand, however, the method has advantages such as no risk of cross-species viral infection derived from the host animal and the indefinite specificity of the antibody (antibodies against forbidden clone, sugar chain and the like can also be easily prepared).
The method of preparing a phage display human antibody library include, but are not limited to, for example, the methods described below.
Although a phage used is not subject to limitation, filamentous phage (Ff bacteriophage) is normally preferably used. As the method of presenting a foreign protein on the phage surface, a method comprising expressing and presenting the foreign protein as a fusion protein with any of the coat proteins g3p, and g6p to g9p on the coat protein can be mentioned; and a method comprising fusing the foreign protein to the N-terminal side of g3p or g8p is often used. As the phage display vector, besides 1) one in which the foreign gene is introduced in the form of fusion gene with the coat protein gene of the phage genome, to allow all the coat proteins presented on the phage surface to be presented as a fusion protein with the foreign protein, 2) one in which the gene encoding the fusion protein is inserted separately from the wild-type coat protein gene to allow the fusion protein and the wild-type coat protein to be expressed simultaneously, and 3) an *E. coli* having a phagemid vector harboring the gene that encodes the fusion protein is infected with a helper phage having the wild-type coat protein gene to produce phage particles that express the fusion protein and the wild-type coat protein simultaneously, and the like can be mentioned. However, a phage display vector of the type 2) or 3) is used for the preparation of an antibody library, because in the case of 1), the capability of infection is lost when a large foreign protein is fused.
As a specific vector, those described by Holt et al. (Curr. Opin. Biotechnol., 11: 445-449, 2000) can be mentioned as examples. For example, pCES1 (see J. Biol. Chem., 274: 18218-18230, 1999) is an Fab-expressing phagemid vector wherein a DNA encoding the $_\kappa$L chain constant region allocated to downstream of the g3p signal peptide, and a DNA encoding CH3, His-tag, c-myc tag, and the amber stop codon (TAG) followed by the g3p coding sequence, allocated to downstream of the g3p signal peptide, are arranged under the control of one lactose promoter. When this is introduced to an *E. coli* having an amber mutation, Fab is presented onto the g3p coat protein, but when it is expressed in the HB2151 strain and the like, which do not have an amber mutation, a soluble Fab antibody is produced. And as the scFv-expressing phagemid vector, for example, pHEN1 *(*J. Mol. Biol., 222: 581-597, 1991) and the like are used.
Meanwhile as examples of the helper phage, M13-KO7, VCSM13 and the like can be mentioned.
And as another phage display vector, a vector that is designed as a DNA sequence comprising the cysteine-encoding codon is linked to each of the 3' end of the antibody gene and the 5' end of the coat protein gene to express the two genes simultaneously and separately (not in the form of a fusion protein), and to present the antibody onto the coat protein on the phage surface via S-S bonds between the introduced cysteine residues (CysDisplay™ technology of Morphosys Company) and the like, can be mentioned.
As the kind of human antibody library, a naive/non-immunized library, a synthetic library, an immunized library and the like can be mentioned.
The naive/non-immunized library is a library obtained by acquiring the $V_H$ and $V_L$ genes retained by a normal human by RT-PCR, and randomly cloning them into the above-described phage display vector. Normally, mRNA derived from lymphocytes of peripheral blood, bone marrow, tonsil and the like of a normal human, and the like are used as the template. A library prepared by selectively amplifying IgM-derived mRNA in which a class switch due to antigen sensitization is not undergoing, to avoid V gene biases such as clinical history, is particularly called a naive library. Representatively, the library of Cambridge Antibody Technology (see J. Mol. Biol., 222: 581-597, 1991; Nat. Biotechnol., 14: 309-314, 1996), the library of Medical Research Council (see Annu. Rev. Immunol., 12: 433-455, 1994), the library of Dyax Corp. (see J. Biol. Chem., 1999. *(supra);* Proc. Natl. Acad. Sci. USA, 14: 7969-7974, 2000) and the like can be mentioned.
A synthetic library is obtained by selecting a functional particular antibody gene in human B cells, and substituting a portion of antigen-binding region in a V gene fragment, for example, CDR3 and the like, with DNAs encoding a random amino acid sequence of appropriate length, to construct a library. It is recognized to be excellent in antibody expression efficiency and stability because the library can be constructed with the combination of the $V_H$ and $V_L$ genes, which produce functional scFv and Fab, since the beginning. Representatively, the HuCAL library of Morphosys AG (see J. Mol. Biol., 296: 57-86, 2000), the library of BioInvent (see Nat. Biotechnol., 18: 852, 2000), the library of Crucell (see Proc. Natl. Acad. Sci. USA, 92: 3938, 1995; J. Immunol. Methods, 272: 219-233, 2003) and the like can be mentioned.
An immunized library is a library obtained by preparing an mRNA from lymphocytes collected from a human such as a patient with cancer, autoimmune disease, infectious disease and the like or a recipient of vaccination, having an elevated blood antibody titer against the target antigen, or from human lymphocytes and the like which are artificially immunized with the target antigen by the above-described in vitro immunization method, in the same manner as with the above-described naive/non-immunized library, and amplifying the $V_H$ and $V_L$ genes by RT-PCR, to construct a library. It is possible to obtain the desired antibody even from such libraries of relatively small size because the desired antibody gene is contained in the library already at the beginning.

The wider the diversity of the library is, the better; actually, however, an appropriate library size is about $10^8$ to $10^{11}$ clones, taking into consideration of the number of phages handlable in the following panning operation ($10^{11}$ to $10^{13}$ phages) and the number of phages necessary to isolate and amplify clones in ordinary panning (100 to 1,000 phages/clone),; it is possible to screen for an antibody normally having a Kd value on the order of $10^{-9}$ with a library of about $10^8$ clones.

The process for selecting an antibody against the target antigen by the phage display method is referred to as panning. To be specific, for example, a phage presenting an antigen-specific antibody is concentrated by repeating a series of operations of bringing an antigen-immobilized carrier and a phage library into contact with each other, washing out the unbound phage, thereafter eluting the bound phage from the carrier, and infecting the phage to E. coli to proliferate it, about 3 to 5 times. As the carrier for immobilizing the antigen, various carriers used in ordinary antigen-antibody reactions or affinity chromatography, for example, insoluble polysaccharides such as agarose, dextran, and cellulose, synthetic resins such as polystyrene, polyacrylamide, and silicon, or microplates, tubes, membranes, columns, beads and the like comprising glass, metal and the like, and surface plasmon resonance (SPR) sensor chips, and the like can be mentioned. For the antigen immobilization, physical adsorption may be used, and a method using a chemical bond used to insolubilize and immobilize a protein or enzyme and the like is also acceptable. For example, a biotin-(strept) avidin system and the like are preferably used. When the endogenous ligand, that is a target antigen, is a small molecule such as a peptide, it is necessary to pay special attention to prevent masking of the portion used as the epitope by conjugating with the carrier. For washing the unbound phage, a blocking solution such as BSA solution (once or twice), a PBS containing a surfactant such as Tween (3 to 5 times) and the like can be used. There is also a report mentioning that the use of citrate buffer (pH 5) and the like is preferable for the washing. For elution of the specific phage, an acid (e.g., 0.1 M hydrochloric acid and the like) is normally used; cleavage with a specific protease (for example, a gene sequence that encodes the trypsin cleavage site can be introduced into the linkage site between the antibody gene and the coat protein gene. In this case, *E. coli* infection and proliferation are possible even if all the coat protein is expressed in the form of a fusion protein because the wild-type coat protein is presented on the surface of the eluted phage), competitive elution with a soluble antigen, or elution by reduction of S-S bond (for example, in the aforementioned CysDisplay™, the antigen-specific phage can be recovered by dissociating the antibody and the coat protein by using a suitable reducing agent after performing panning.) is also possible. When elution has been performed with an acid, the eluate is neutralized with Tris and the like, and the eluted phage is then infected to *E. coli*, which is cultured; after which the phage is recovered by a conventional method.

After the phage presenting the antigen-specific antibody is concentrated by panning, the phage is infected to *E. coli* and the cells are sown onto a plate to perform cell cloning. The phage is again collected from the each clone, and the antigen binding activity is confirmed by the above-described antibody titer assay (e.g., ELISA, RIA, FIA and the like) or a measurement utilizing FACS or SPR.

Isolation and purification of the antibody from the selected phage clone that presents the antigen-specific antibody can be performed by, for example, when using a vector incorporating an amber stop codon at the linker site of the antibody gene and the coat protein gene as the phage display vector, infecting the phage to an *E. coli* that does not have amber mutation (e.g., HB2151 strain) to produce and secrete soluble antibody molecules in periplasm or the medium, lysing the cell wall with lysozyme and the like, collecting the extracellular fraction, and purifying using the same purification technique as described above. Provided that the His-tag or c-myc tag has been introduced in advance, the antibody can easily be purified by using IMAC, an anti-c-myc antibody column and the like. When cleavage with a specific protease is utilized in panning, the antibody molecule is separated from the phage surface by an action with the protease, so that the desired antibody can be purified by performing the same purification operation as above mentioned.

Confirmation of the thus-obtained antibody to be a non-neutralizing antibody can be achieved in the same manner as with the above-described heterologous monoclonal antibody.

The technology for producing a fully human antibody using a human antibody-producing animal and a phage display human antibody library can also be applied to the production of a monoclonal antibody derived from another animal species. For example, animals widely propagated as domestic animals (domestic fowls) such as bovine, swine, sheep, goat, and chicken, and pet animals such as dogs and cats, and the like can be mentioned as the subject animal species. In non-human animals, the utilization of an immunized library is more effective because there are fewer ethical problems concerning artificial' immunization with the target antigen.

The non-neutralizing antibody (preferably a monoclonal antibody) against an endogenous ligand, obtained as described above, can be used as an agent for improving the blood stability of the endogenous ligand, after being mixed with a pharmacologically acceptable carrier into a pharmaceutical composition, as required.

As the pharmacologically acceptable carrier here, various organic or inorganic carrier substances conventionally used as materials to prepare medicine can be used, which is compounded into as excipient, solvent (dispersing agent), solubilizer, suspending agent, stabilizer, isotonizing agent, buffer, pH adjusting agent, soothing agent and the like. Where necessary, preparation additives such as preservatives, antioxidants and the like can also be used.

As examples of preferable excipients, lactose, sucrose, D-mannitol, D-sorbitol, starch, α-starch, dextrin, crystalline cellulose, low-substituted hydroxypropylcellulose, carboxymethylcellulose sodium, gum arabic, pullulan, light silicic anhydride, synthetic aluminum silicate, magnesium metasilicate aluminate and the like can be mentioned.

As examples of preferable solvents, water for injection, physiological saline, Ringer's solution, alcohol, propylene glycol, polyethylene glycol, sesame oil, corn oil, olive oil, cottonseed oil and the like can be mentioned.

As examples of preferable solubilizers, polyethylene glycol, propylene glycol, D-mannitol, trehalose, benzyl benzoate, ethanol, trisaminomethane, cholesterol, triethanolamine, sodium carbonate, sodium citrate, sodium salicylate, sodium acetate and the like can be mentioned.

As examples of preferable suspending agents, surfactants such as stearyltriethanolamine, sodium lauryl sulfate, laurylaminopropionic acid, lecithin, benzalkonium chloride, benzethonium chloride, and glyceryl monostearate; hydrophilic polymers such as polyvinyl alcohol, polyvinylpyrrolidone, carboxymethylcellulose sodium, methylcellulose, hydroxymethylcellulose, hydroxyethylcellulose, and hydroxypropylcellulose; polysorbates, polyoxyethylene hydrogenated castor oil and the like can be mentioned.

As examples of preferable stabilizers, human serum albumin (HSA), sodium pyrosulfite, Rongalit, sodium metahydrogensulfite and the like can be mentioned.

As examples of preferable isotonizing agents, sodium chloride, glycerin, D-mannitol, D-sorbitol, glucose and the like can be mentioned.

As examples of preferable buffers, buffer solutions such as of phosphates, acetates, carbonates and citrates, and the like can be mentioned.

As examples of preferable pH adjusting agent, acid or base such as hydrochloride, sodium hydroxide and the like can be mentioned.

As examples of preferable soothing agents, benzyl alcohol and the like can be mentioned.

As examples of preferable preservatives, p-hydroxybenzoates, chlorobutanol, benzyl alcohol, phenethyl alcohol, dehydroacetic acid, sorbic acid and the like can be mentioned.

As examples of preferable antioxidants, sulfites, ascorbic acid salts and the like can be mentioned.

As examples of dosage forms for the aforementioned pharmaceutical composition, injection type preparations such as injections (e.g., subcutaneous injections, intravenous injections, intramuscular injections, intraperitoneal injections, intraarterial injections and the like), drip infusions and the like can be mentioned.

These pharmaceutical compositions can be produced by a method in common use in the field of drug formulation technology, for example, methods described in the Japanese Pharmacopoeia and the like. Specific methods of preparing preparations are described in detail below. The antibody content in the pharmaceutical composition varies depending on dosage form, antibody dose and the like, and is, for example, about 0.1% to 100% by weight.

For example, an injection is produced by dissolving, suspending or emulsifying antibody, along with a dispersing agent (e.g., polysorbate 80, polyoxyethylene hydrogenated castor oil 60, polyethylene glycol, carboxymethylcellulose, sodium alginate and the like), a preservative (e.g., methylparaben, propylparaben, benzyl alcohol, chlorobutanol, phenol and the like), an isotonizing agent (e.g., sodium chloride, glycerin, D-mannitol, D-sorbitol, glucose and the like) and the like, in an aqueous solvent (e.g., distilled water, physiological saline, Ringer's solution and the like) or an oily solvent (e.g., vegetable oils such as olive oil, sesame oil, cottonseed oil and corn oil, propylene glycol and the like). If desired, additives such as a solubilizer (e.g., sodium salicylate, sodium acetate and the like), a stabilizer (e.g., human serum albumin and the like), and a soothing agent (e.g., benzyl alcohol and the like) may be used. The injection is subjected, for example, to a sterilization treatment as necessary, such as sterilization by filtration using a membrane filter etc., and usually filled in a suitable container such as ampoule etc..

The injection can also be used as a fresh supply obtained by dissolving (dispersing) a powder prepared by treating the above-described liquid by vacuum drying and the like. As examples of the vacuum drying method, lyophilization, a method using the Speedback Concentrator (SAVANT Company), and the like can be mentioned. When performing lyophilization, it is preferable to lyophilize the sample, cooled below -10°C, using a flask in the laboratory or a tray or vial in industrial settings. When the Speedback Concentrator is used, lyophilization is performed at about 0 to 30°C under a vacuum of about 20 mmHg or less, preferably about 10 mmHg or less. It is preferable to add a buffering agent such as a phosphate to the liquid to be dried, to obtain a pH of about 3 to 10. The powder preparation obtained by lyophilization, as a long-stable preparation, can be prepared freshly as an injection by dissolving in water for injection, saline, Ringer's solution and the like, or by dispersing in olive oil, sesame oil, cottonseed oil, corn oil, propyleneglycol and the like before use.

Because the agent for improving the blood stability of the present invention, which is obtained as above, is safe and less toxic, it can be administered to, for example, mammals (for example, humans, rats, rabbits, sheep, swine, cattle, cats, dogs, monkeys and the like) subcutaneously, intravenously, intramuscularly, intraperitoneally, intraarterially or intraventricularly.

The dose of the agent for improving the blood stability of the present invention is not subject to limitation, as long as it is an amount enabling an increase in the blood concentration of an endogenous ligand in the recipient mammal to a range effective for the treatment or prophylaxis for a disease the animal has contracted or can contract, and/or enabling an extension of the blood half-life of the endogenous ligand to an extent effective for the treatment or prophylaxis for the disease. For example, when the agent for improving the blood stability of the present invention is subcutaneously administered to a human for the purpose of improving the blood stability of endogenous GLP-1, the minimum required concentration in blood of GLP-1 and a functional fragment thereof is estimated to be about 100 pM in total. If we assume that a 10- to 100-fold amount of anti-GLP-1 antibody is necessary to form an immune complex with all blood GLP-1, a required blood GLP-1 concentration can be achieved by administering about 1 to 10 nM or, in terms of weight, about 10 to 100 μg/kg body weight, per dosing. This amount can be administered at intervals of, for example, one to several weeks.

However, the dose also varies depending on the normal blood concentration and blood half-life of the target endogenous ligand, stability of the antibody molecule, tissue transfer of the ligand-antibody complex, antigen affinity of the antibody, neutralization activity of the antibody, target disease, severity, in addition, animal species, age, route of administration and the like. Therefore, the general range of the dose of the agent for improving the blood stability of the present invention is even wider; for example, about 0.1 μg to 10 mg/kg, preferably about 1 μg to 1 mg/kg, per dosing can be used.

Preferably, when the agent for improving the blood stability of the present invention is administered to a mammal, the blood half-life of the complex of the endogenous ligand and the antibody is extended more than about 2 times, more preferably more than about 10 times, particularly preferably more than about 50 times, compared with that in the case of the endogenous ligand alone (i.e., free form). Such an elongation effect results in a blood concentration of the endogenous ligand with administration of the agent for improving the blood stability of the present invention to a mammal, more than about 2 times, more preferably more than about 5 times, particularly preferably more than about 10 times, higher than the blood concentration obtained without administering the preparation. As mentioned herein, a blood concentration is measured at an optionally chosen time between a dosing and the subsequent dosing.

Because the agent for improving the blood stability of the present invention can stabilize an endogenous ligand by the action of the antibody which is the active ingredient of the preparation, as described above, and also because the antibody substantially does not neutralize the antigen endogenous ligand, the agent of the present invention can be used as a prophylactic or therapeutic agent for a disease for which enhancing the receptor activity-regulatory action of the ligand by increasing the blood concentration of the endogenous ligand and/or extending the blood half-life is prophylactically or therapeutically effective.

Examples of such diseases include, but are not limited to, metabolic disease, bone and joint disease, cardiovascular disease, cranial/nerve disease, infectious disease, cancer, blood disorder, urologic disease, infertility/erectile dysfunction, deficient growth and immunodeficiency and the like.

Describing in more detail the target disease in relation to association with individual endogenous ligands serving as target antigens, examples of the metabolic disease include diabetes (target ligand: GLP-1, calcitonin, PACAP, VIP, insulin, beta-cellulin, beta-cellulin-δ4 and the like), obesity. (target ligand: leptin, adiponectin, GPR7/GPR8 ligand (NPW), MSH and the like), anorexia (target ligand: ghrelin and the like) and the like; examples of the bone and joint disease include osteoporosis (target ligand: calcitonin, PTH and the like) and the like; examples of the cardiovascular disease include ischemic heart diseases (myocardial infarction, angina pectoris) (target ligand: ghrelin, adrenomedullin and the like), hypertension (target ligand: ANP, BNP, CNP and the like) and the like; examples of the cranial nerve disease include ischemic cranial neuropathy (target ligand: ghrelin, VIP and the like), Alzheimer's disease (target ligand: LH-RH and the like) and the like; examples of the infectious disease include viral infectious disease (target ligand: interferon, GM-CSF and the like) and the like; examples of the cancer include prostate cancer, breast cancer (target ligand: LH-RH and the like), primary carcinoma or metastatic carcinoma of various organs (target ligand: metastin, interferon, TNF, IL-2, M-CSF, GM-CSF and the like) and the like; examples of the blood disorder include anemia (target ligand: EPO, GM-CSF and the like) and the like; examples of the urologic disease include dysuria (target ligand: ANP, BNP, CNP and the like) and the like; examples of the infertility/erectile dysfunction include infertility (target ligand: metastin and the like), erectile dysfunction (target ligand: VIP and the like) and the like; examples of the deficient growth include growth hormone deficient short statue (hyperphysical dwarf), Turner's syndrome, Prader-Willi syndrome (target ligand: growth hormone) and the like; examples of the immunodeficiency include HIV infection, after immunosuppressant administration at the time of transplantation or treatment for autoimmune disease (target ligand: GM-CSF and the like) and the like.

When amino acid etc. are to be indicated with abbreviations in this specification, the abbreviations are adopted from IUPAC-IUB Commission on Biochemical Nomenclature or those commonly used in the art. For example, the following abbreviations are used. When an optical isomer is capable of existing with respect to the amino acids, the L-form is represented unless otherwise specified.

PAM : Phenylacetamidomethyl

BHA : Benzhydrylamine
Boc : t-Butyloxycarbonyl
C1-Z: 2-Chloro-benzyloxycarbonyl
Br-Z: 2-Bromo-benzyloxycarbonyl
Bzl : Benzyl
OBzl: Benzyl ester
Tos : p-Toluenesulfonyl
HOBt: 1-Benzotriazole
DCC : N,N'-Dichlorohexylcarbodiimide
Gly : Glycine
Ala : Alanine
Val : Valine
Leu : Leucine
Ile : Isoleucine
Ser : Serine
Thr : Threonine
Cys : Cysteine
Met : Methionine
Glu : Glutamic acid
Asp : Aspartic acid
Lys : Lysine
Arg : Arginine
His : Histidine
Phe : Phenylalanine
Tyr : Tyrosine
Trp : Tryptophan
Pro : Proline
Asn : Asparagine
Gln : Glutamine

The present invention is explained in detail in the following by referring to Examples, which are mere exemplifications and not to be construed as limiting the scope of the present invention.

The anti-PACAP antibody-producing hybridoma obtained in the Examples below has been deposited with the Fermentation Research Institute, the Agency of Industrial Science and Technology, the Ministry of International Trade and Industry, Japan (then) [now the International Patent Organism Depositary (IPOD), National Institute of Advanced Industrial Science and Technology (Central 6, 1-1-1, Higashi, Tsukuba, Ibaraki, 305-8566 Japan)] under the following accession numbers since March 16, 1990:

hybridoma cell
PA-1N:FERM BP-2811
PA-3N:FERM BP-2812
PA-5N:FERM BP-2813
PA-6N:FERM BP-2814
PA-2C:FERM BP-2815
PA-1C:FERM BP-2816

In the Examples below, antibodies obtained from each hybridoma cell are denoted with an "a" after the name of the cell (for example, antibody obtained from PA-1N cell is PA-1Na).

In addition, hybridoma (GLIT2-329(1)24) producing anti-GLP-1 non-neutralizing antibody GLIT2-329(1)24 was accepted as of March 17, 2005 by the IPOD (mentioned above) under the accession No. of FERM ABP-10297.

**Reference Example 1**

(1) Synthesis of PACAP38NH$_2$

PACAP38NH$_2$ (SEQ ID NO:1)was synthesized by using 1.04 g (0.5 mmole) of a commercially available p-methyl BHA resin (Applied Biosystems Inc.) and a peptide synthesizer (Model 430A, Applied Biosystems Inc.).

A starting amino acid, Boc-Lys(C1-Z), was activated with HOBt/DCC and then condensed to the resin. Thereafter, the Boc group on the resin was treated with 50% trifluoroacetic acid/methylene chloride to deprotect the amino group. To this free amino group, the following protected amino acids activated with HOBt/DCC were condensed in turn according to the amino acid sequence of PACAP38NH$_2$:

Boc-Asn, Boc-Lys(Cl-Z), Boc-Val, Boc-Arg(Tos), Boc-Gln, Boc-Tyr(Br-Z), Boc-Gly, Boc-Leu, Boc-Ala, Boc-Met, Boc Ser(Bzl), Boc-Asp(OBzl), Boc-Thr(Bzl), Boc-Phe, Boc Ile, and Boc-His(Tos). After the additional condensation by the same amino acid derivatives activated by DCC or HOBt/DCC, the unreacted amino groups were acetylated with acetic anhydride to obtain 2.42 g of a protected PACAP38NH$_2$ resin.

0.51 g of the resulting protected PACAP38NH$_2$ resin was treated with 5 ml of hydrogen fluoride in the presence of 0.6 g of p-cresol at 0˚C for 60 minutes, followed by removal of excess hydrogen fluoride by distillation under reduced pressure. The residue was washed twice with 5 ml of ethyl ether, and then extracted with 6 ml of 50% aqueous acetic acid. The insoluble material was removed by filtration and washed with 5 ml of 50% aqueous acetic acid. The filtrate and the washings were combined, and the combined solution was concentrated to 2 to 3 ml under reduced pressure. The concentrated solution was applied on a Sephadex LH-20 column (2 X 90 cm), and eluted with 50% acetic acid. The main fractions were collected, followed by removal by distillation under reduced pressure. Then, the residue was dissolved in 100 ml of 0.1% aqueous trifluoroacetic acid. The resulting solution was applied on a YMC-ODS AM120 S-50 resin column (1.6 X 7 cm) and eluted by a linear concentration gradient of between 0.1% aqueous trifluoroacetic acid and 50% acetonitrile (containing 0.1% trifluoroacetic acid).

The main fractions were combined and lyophilized. Thus, 60 mg of white powder was obtained. This powder was dissolved in 20 ml of 0.05 M aqueous ammonium acetate. The resulting solution was applied on a CM-Cellulofine resin column (1 X 6 cm) and eluted by a linear concentration gradient of from 0.05 M to 1 M ammonium acetate. The main fractions were combined and applied on a YMC-ODS column (2.6 X 7 cm) again and eluted by a linear gradient of from 0% to 40% aqueous acetonitrile (containing 0.1% trifluoroacetic acid). The elution fractions containing 28% to 30% acetonitrile were collected and lyophilized. Thus, 21.6 mg of white powder was obtained.

Analytical values of amino acid composition:

Asp 2.90(3), Thr 0.84(1), Ser 2.10(3), Glu 2.21(2), Gly 2.00(2), Ala 3.29(3), Val 3.19(3), Met 1.01(1), Ile 0.87(1), Leu 2.09(2), Tyr 3.94(4), Phe 0.92(1), Lys 7.18(7), His 0.96(1), Arg 4.19(4) $(M + H)^+$ by a mass spectrography: 4530 HPLC elution time: 19.6 minutes Column conditions Column: YMC-ODS (AM-301, S-5 120A) Eluent: A (0.1% aqueous trifluoroacetic acid) B (acetonitrile containing 0.1% trifluoroacetic acid) A linear concentration gradient elution from the eluent A to the eluent B (50 minutes) Flow rate: 1.0 ml/minute

(2) Synthesis of PACAP27NH$_2$

PACAP27NH$_2$ (SEQ ID NO:2) was synthesized by using 1.04 g (0.5 mmole) of a commercially available p-methyl BHA resin (Applied Biosystems Inc.) and a peptide synthesizer (Model 430A, Applied Biosystems Inc.).
A starting amino acid, Boc-Leu, was activated with HOBt/DCC and then condensed to the resin. Thereafter, the Boc group on the resin was treated with 50% trifluoroacetic acid/methylene chloride to deprotect the amino group. To this free amino group, the following protected amino acids activated with HOBt/DCC were condensed in turn according to the amino acid sequence of PACAP27NH$_2$:

Boc-Lys(Cl-Z), Boc-Val, Boc-Arg(Tps), Boc-Gln, Boc-Tyr(Br-Z), Boc-Gly, Boc-Leu, Boc-Ala, Boc-Met, Boc-Ser(Bzl), Boc Asp(OBzl), Boc-Thr(Bzl), Boc-Phe, Boc-Ile, and Boc His(Tos). After the additional condensation by the same amino acid derivatives activated by DCC or HOBt/DCC, the unreacted amino groups were acetylated with acetic anhydride to obtain 2.31 g of a protected PACAP27NH$_2$ resin.

0.79 g of the resulting protected PACAP27NH$_2$ resin was treated with 10 ml of absolute hydrogen fluoride in the presence of 1.2 g of p-cresol at 0˚C for 60 minutes, followed by removal of excess hydrogen fluoride by distillation under reduced pressure. The residue was washed twice with 5 ml of ethyl ether, and then extracted with 5 ml of 50% aqueous acetic acid. The insoluble material was removed by filtration and washed with 5 ml of 50% aqueous acetic acid. The filtrate and the washings were combined, and the combined solution was concentrated to 2 to 3 ml under reduced pressure. The concentrated solution was applied on a Sephadex LH-20 column (2 X 75 cm) for elution with 50% acetic acid. The main fractions were collected, followed by distillation under reduced pressure. The residue was dissolved in 100 ml of 0.1% aqueous trifluoroacetic acid. The resulting solution was applied on a YMC-ODS AM120 S-50 resin column (2.6 X 7 cm) and eluted by a liner concentration gradient of between 0.1% aqueous trifluoroacetic acid and 50% acetonitrile (containing 0.1% trifluoroacetic acid). The main fractions were combined and was applied onto a YMC-ODS column (2.6 X 7 cm) again and eluted by a linear concentration gradient of from 15 to 35% aqueous acetonitrile solution (containing 0.1% trifluoroacetic acid). The 30 to 32% fractions of acetonitrile were collected and lyophilized. The resulting product was dissolved in 20 ml of 0.05M-aqueous ammonium acetate. The solution was applied onto a CM Cellulofine resin column (1 X 6 cm) and eluted by a linear concentration gradient of water to 0.33 M - aqueous ammonium acetate. The main fractions (0.18 to 0.22 M) were collected and lyophilized. Thus, 20 mg of white powder was obtained.

Analytical values for amino acid composition:

Asp 1.96(2), Thr 0.94(1), Ser 2.57(3), Glu 1.07(1),
Gly 0.95(1), Ala 3.00(3), Val 1.96(2), Met 0.88(1),
Ile 0.88(1), Leu 1.93(2), Tyr 2.87(3), Phe 0.90(1),
Lys 2.91(3), His 0.94(1), Arg 2.17(2)
$(M+H)^+$ by a mass spectrography: 3146.7
HPLC elution time: 21.2 minutes
Column conditions
Column: YMC-ODS (AM-301, S-5 120A)
Eluent: A (0.1% aqueous trifluoroacetic acid)
B (acetonitrile containing 0.1% trifluoroacetic acid)
A linear concentration gradient elution from the eluent A to the eluent B (50 minutes)
Flow rate: 1.0 ml/minute

(3) Synthesis of PACAP (14-38) $NH_2$

PACAP(14-38)$NH_2$ (SEQ ID NO:3) was synthesized by using 1.04 g (0.5 mmole) of a commercially available p-methyl BHA resin (Applied Biosystems Inc.) and a peptide synthesizer (Model 430A, Applied Biosystems Inc.).
A starting amino acid, Lys(Cl-Z), was activated with HOBt/DCC and then condensed to the resin. Thereafter, the Boc group on the resin was treated with 50% trifluoroacetic acid/methylene chloride to deprotect the amino group. To this free amino group, the following protected amino acids activated with HOBt/DCC were condensed in turn according to the amino acid sequence of PACAP (14-38) $NH_2$:

Boc-Asn, Boc-Lys(C1-Z), Boc-Val, Boc-Arg(Tos), Boc-Gln,
Boc-Tyr(Br-Z), Boc-Gly, Boc-Leu, Boc-Ala, Boc-Met. After the additional condensation by the same amino acid derivatives activated by DCC or HOBt/DCC, the unreacted amino groups were acetylated with acetic anhydride to obtain 2.00 g of a protected PACAP (14-38) $NH_2$ resin.

0.48 g of the resulting protected PACAP(14-38)$NH_2$ resin was treated with 5 ml of absolute hydrogen fluoride in the presence of 0.48 g of p-cresol at 0°C for 60 minutes, followed by removal of excess hydrogen fluoride by distillation under reduced pressure. The residue was washed twice with 5 ml of ethyl ether, and then extracted with 5 ml of 50% aqueous acetic acid. The insoluble material was removed by filtration and washed with 5 ml of 50% aqueous acetic acid. The filtrate and the washings were combined, and the combined solution was concentrated to 2 to 3 ml under reduced pressure. The concentrated solution was applied on a Sephadex LH-20 column (2 X 75 cm) and eluted with 50% acetic acid. The main fractions were collected, followed by distillation under reduced pressure. The residue was dissolved in 100 ml of 0.1% aqueous trifluoroacetic acid. The resulting solution was applied onto a YMC-ODS AM120 S-50 resin column (2.6 X 7 cm) and eluted by a liner concentration gradient of between 0.1% aqueous trifluoroacetic acid and 30% acetonitrile (containing 0.1% trifluoroacetic acid). The main fractions were collected and lyophilized. Thus, 20.2 mg of white powder was obtained.
Analytical values for amino acid composition:

Asp 1.01(1), Glu 2.01(2), Gly 1.00(1), Ala 3.01(3),
Val 2.85(3), Met 0.86(1), Leu 2.08(2), Tyr 1.98(2),

Lys 6.37(7), Arg 3.24(3)

$(M + H)^+$ by a mass spectrography: 3003.6

HPLC elution time: 13.1 minutes

Column conditions

Column: YMC-ODS (AM-301, S-5 120A)

Eluent: A (0.1% aqueous trifluoroacetic acid)

B (acetonitrile containing 0.1% trifluoroacetic acid)

A linear concentration gradient elution from the eluent A to the eluent B (25 minutes)

Flow rate: 1.0 ml/minute

(4) Synthesis of PACAP (1-13) OH

PACAP(1-13)OH (SEQ ID NO:4) was synthesized by using 0.87 g (0.5 mmole) of a commercially available Boc-Tyr(Br-Z)-OCH$_2$-PAM resin (Applied Biosystems Inc.) and a peptide synthesizer (Model 430A, Applied Biosystems Inc.).

The Boc group on the resin was treated with 50% trifluoroacetic acid/methylene chloride to deprotect the amino group. To this free amino group, the following protected amino acids activated with HOBt/DCC were condensed in turn according to the amino acid sequence of PACAP(1-13)OH:

Boc-Arg (Tos), Boc-Tyr(Br-Z), Boc-Gly, Boc-Ser(Bzl),

Boc-Asp(OBzl), Boc-Thr(Bzl), Boc-Phe, Boc-Ile, and

Boc-His(Tos). After the additional condensation by the same amino acid derivatives activated by DCC or HOBt/DCC, the unreacted amino groups were acetylated with acetic anhydride to obtain 1.86 g of a protected PACAP(1-13) OH$_2$-PAM resin.

0.70 g of the resulting protected resin was treated with 10 ml of absolute hydrogen fluoride in the presence of 0.81 g of p-cresol at 0°C for 60 minutes, followed by removal of excess hydrogen fluoride by distillation under reduced pressure. The residue was washed twice with 5 ml of ethyl ether, and then extracted with 5 ml of 50% aqueous acetic acid. The insoluble material was removed by filtration and washed with 5 ml of 50% aqueous acetic acid. The filtrate and the washings were combined, and the combined solution was concentrated to 2 to 3 ml under reduced pressure. The concentrated solution was applied onto a Sephadex LH-20 column (2 X 75 cm) for elution with 50% acetic acid. The main fractions were collected, followed by distillation under reduced pressure. The residue was dissolved in 100 ml of 0.1% aqueous trifluoroacetic acid. The resulting solution was applied onto a YMC-ODS AM120 S-50 resin column (2.6 X 7 cm) and eluted by a liner concentration gradient of between 0.1% aqueous trifluoroacetic acid and 33% acetonitrile (containing 0.1% trifluoroacetic acid). The main fractions were combined and the combined solution was purified again under the same column conditions. The main fractions were collected and lyophilized. Thus, 38 mg of white powder was obtained.

Analytical values for amino acid composition:

Asp 2.00(2), Thr 0.93(1), Ser 2.43(3), Glu 1.05(1),

Gly 1.00(1), Tyr 1.82(2), Phe 1.02(1), His 1.31(1),

Arg 1.12(1)

$(M +H)^+$ by a mass spectrography: 1547.5

HPLC elution time: 12.3 minutes

Column conditions

Column: YMC-ODS (AM-301, S-5 120A)

Eluent: A (0.1% aqueous trifluoroacetic acid)

B (acetonitrile containing 0.1% trifluoroacetic acid)

A linear concentration gradient elution from the eluent A to the eluent B (25 minutes)

Flow rate: 1.0 ml/minute

(5) Synthesis of PACAP(4-27)OH

PACAP(4-27)OH (SEQ ID NO:5) was synthesized by using 0.60 g (0.5 mmole) of a commercially available Boc-Leu-OCH$_2$-PAM resin (Applied Biosystems Inc.) and a peptide synthesizer (Model 430A, Applied Biosystems Inc.).

The Boc group on the resin was treated with 50% trifluoroacetic acid/methylene chloride to deprotect the amino group. To this free amino group, the following protected amino acids activated with HOBt/DCC were condensed in turn according to the amino acid sequence of PACAP(4-27)OH:

Boc-Lys(Cl-Z), Boc-Val, Boc-Arg(Tos), Boc-Gln,

Boc-Tyr(Br-Z), Boc-Gly, Boc-Leu, Boc-Ala, Boc-Met,

Boc-Ser(Bzl), Boc-Asp(OBzl), Boc-Thr(Bzl), Boc-Phe,

Boc-Ile. After the additional condensation by the same amino acid derivatives activated by DCC or HOBt/DCC, the unreacted amino groups were acetylated with acetic anhydride to obtain 1.08 g of a protected PACAP(4-27) $OCH_2$-PAM resin.

0.29 g of the resulting protected PACAP(4-27)$OCH_2$-PAM resin was treated with 5 ml of absolute hydrogen fluoride in the presence of 0.49 g of p-cresol at 0°C for 60 minutes, followed by removal of excess hydrogen fluoride by distillation under reduced pressure. The residue was washed twice with 5 ml of ethyl ether, and then extracted with 5 ml of 50% aqueous acetic acid. The insoluble material was removed by filtration and washed with 5 ml of 50% aqueous acetic acid. The filtrate and the washings were combined, and concentrated to 2 to 3 ml under reduced pressure. The concentrated solution was applied on a Sephadex LH-20 column (2 X 75 cm) and eluted with 50% acetic acid. The main fractions were collected, followed by distillation under reduced pressure. The residue was dissolved in 100 ml of 0.1% aqueous trifluoroacetic acid. The resulting solution was applied onto a YMC-ODS AM120 S-50 resin column (2.6 X 7 cm) and eluted by a liner concentration gradient of between 15% acetonitrile (containing 0.1% trifluoroacetic acid) and 50% acetonitrile (containing 0.1% trifluoroacetic acid). The main fractions were collected, and lyophilized to obtain 33 mg of white powder. The powder was dissolved in 20 ml of 0.05M-aqueous ammonium acetate. The solution was applied onto a CM-Cellurofine resin column (1 x 6 cm) and eluted by a linear concentration gradient with water to 0.30M-aqueous ammonium acetate. The main fractions (0.18 to 0.22 M) were collected, and lyophilized. Thus, 33 mg of white powder was obtained.

Analytical values for amino acid composition:

Asp 1.02(1), Thr 0.98(1), Ser 1.78(2), Glu 1.07(1),

Gly 1.02(1), Ala 3.04(3), Val 1.89(2), Met 0.81(1),

Ile 0.89(1), Leu 2.00(2), Tyr 2.91(3), Phe 0.90(1),

Lys 2.89(3), Arg 2.20(2)

$(M + H)^+$ by a mass spectrography: 2808.5

HPLC elution time: 14.5 minutes

Column conditions

Column: YMC-ODS (AM-301, S-5 120A)

Eluent: A (0.1% aqueous trifluoroacetic acid)

B (acetonitrile containing 0.1% trifluoroacetic acid)

A linear concentration gradient elution from the eluent A to the eluent B (35 minutes)

Flow rate: 1.0 ml/minute

(6) Synthesis of PACAP (31-38) $NH_2$

PACAP(31-38)$NH_2$ (SEQ ID NO:6) was synthesized by using 0.98 g (0.5 mmole) of a commercially available p-methyl BHA resin (Applied Biosystems Inc.) and a peptide synthesizer (Model 430A, Applied Biosystems Inc.).

A starting amino acid, Boc-Lys(Cl-Z), was activated with HOBt/DCC and then condensed to the resin. Thereafter, the Boc group on the resin was treated with 50% trifluoroacetic acid/methylene chloride to deprotect the amino group. To this free amino group, the following protected amino acids activated with HOBt/DCC were condensed in turn according to the amino acid sequence of PACAP(31-38)$NH_2$:

Boc-Asn, Boc-Lys(Cl-Z), Boc-Val, Boc-Arg(Tos), Boc-Gln, Boc-Tyr (Br-Z). After the additional condensation by the same amino acid derivatives activated by DCC or HOBt/DCC, the unreacted amino groups were acetylated with acetic anhydride to obtain 2.00 g of a protected PACAP(31-38)$NH_2$ resin.

0.43 g of the resulting protected PACAP(31-38)$NH_2$ resin was treated with 5 ml of absolute hydrogen fluoride in the presence of 0.6 g of p-cresol at 0°C for 60 minutes, followed by removal of excess hydrogen fluoride by distillation under reduced pressure. The residue was washed twice with 5 ml of ethyl ether, and then extracted with 5 ml of 50% aqueous acetic acid. The insoluble material was removed by filtration and washed with 5 ml of 50% aqueous acetic acid. The filtrate and the washings were combined, and concentrated to 2 to 3 ml under reduced pressure. The concentrated solution was applied on a Sephadex LH-20 column (2 X 75 cm) and eluted with 50% acetic acid. The main fractions were collected, followed by distillation under reduced pressure. The residue was dissolved in 100 ml of 0.1% aqueous trifluoroacetic acid. The resulting solution was applied onto a YMC-ODS AM120 S-50 resin column (2.6 X 7 cm) and eluted by a liner concentration gradient of 0.1% aqueous trifluoroacetic acid and 33% acetonitrile (containing 0.1% trifluoroacetic acid). The main fractions were collected, and lyophilized. Thus, 45 mg of white powder was obtained.

Analytical values for amino acid composition:

Asp 1.02(1), Glu 1.05(1), Val 1.00(1), Tyr 0.90(1),
Lys 2.98(3), Arg 1.12(1)
$(M + H)^+$ by a mass spectrography: 1062.7
HPLC elution time: 11.6 minutes
Column conditions
Column: YMC-ODS (AM-301, S-5 120A)
Eluent: A (0.1% aqueous trifluoroacetic acid)
B (acetonitrile containing 0.1% trifluoroacetic acid)
A linear concentration gradient elution from the eluent A to the eluent mixture [A:B(4:1)] (20 minutes)
Flow rate: 1.0 ml/minute

**Example 1**

(1) Preparation of Immunogen Containing PACAP38NH$_2$

A complex comprising PACAP38NH$_2$ obtained in Reference Example 1(1) described above and bovine thyroglobulin (BTG) was prepared, and it was used as an immunogen. Namely, 2.8 mg of PACAP38NH$_2$ and 8.4 mg of BTG were dissolved in 1 ml of 0.1 M phosphate buffer (pH 6.9), and glutaraldehyde was added thereto to a final concentration of 0.04%, followed by reaction at room temperature for 2 hours. After the reaction, the resulting product was dialyzed against saline at 4°C for 2 days.

(2) Immunization of PACAP38NH$_2$-BTG Complex

The female BALB/C mice (6 to 8 weeks old) were subcutaneously immunized with 80 μg/mouse of the immunogen PACAP38NH$_2$-BTG complex, obtained in above (1), together with Freund's complete adjuvant. Then, the mice were additionally immunized with the same amount of the immunogen, together with Freund's incomplete adjuvant, 2 to 3 times at 4-week intervals.

**Example 2**

(1) Preparation of Horseradish Peroxidase (HRP) Labeled PACAP38NH$_2$

A labeled PACAP38NH$_2$ for the enzyme immunoassay (EIA) was prepared by crosslinking PACAP38NH$_2$ obtained in Reference Example 1(1) and HRP. Namely, 180 nmoles of PACAP38NH$_2$ was dissolved in 500 μl of 0.1 M phosphate buffer (pH 6.8), and 50 μl of a DMF solution containing 450 nmoles of GMBS was mixed therewith, followed by reaction at room temperature for 30 minutes. After the reaction, the resulting product was fractionated on a Sephadex G-15 column. Thus, 100 nmoles of a maleimide group-introduced polypeptide was obtained. On the other hand, 7.9 mg (200 nmoles) of HRP was dissolved in 0.95 ml of 0.02 M phosphate buffer (pH 6.8) containing 0.15 M NaCl, and 50 μl of a DMF solution containing 1.54 mg (4.9 μmoles) of [N-succinimidyl-3-(2-pyridylthio)propionate] (SPDP) was mixed therewith, followed by reaction at room temperature for 40 minutes. After the reaction, 0.33 ml of 0.1 M acetate buffer (pH 4.5) containing 8.2 mg (53 μmoles) of dithiothreitol was added thereto, followed by reaction at room temperature for 20 minutes. Then, the reaction product was fractionated on a Sephadex G-25 column. Thus, 6 mg (100 nmoles) of a SH group-introduced enzyme was obtained. Then, 100 nmoles of maleimide group-introduced PACAP38NH$_2$ and 100 nmoles of SH group-introduced HRP were mixed and reacted with each other at 4°C for 16 hours. After the reaction, the reaction product was fractionated on Ultrogel AcA44 (LKB-Pharmacia) to obtain HRP-labeled PACAP38NH$_2$.

(2) Measurement of Antibody Titer of Mouse Antiserum

The antibody titer of the mouse antiserum was measured by the following method. In order to prepare an anti-mouse immunoglobulin antibody-bound microplate, 100 μl of 0.1 M carbonate buffer (pH 9.6) containing 100 μg/ml of the anti-mouse immunoglobulin antibody [IgG fraction, Kappel] was first poured into each well of a 96-well microplate, and the plate was allowed to stand at 4°C for 24 hours. After the plate was washed with PBS, 300 μl of PBS containing 25% Blockace (Snow Brand Milk Products) was poured into each well to block excess binding sites of the wells, and treated at a temperature of 4°C for at least 24 hours. To each well of the above-mentioned anti-mouse immunoglobulin antibody-bound microplate, 50 μl of buffer E [0.02 M phosphate buffer (pH 7.0) containing 10% Blockace, 2 mg/ml bovine serum albumin (BSA), 0.4 M NaCl, 2 mM EDTA and 0.1% NaN$_3$] or 50 μl of the mouse anti-PACAP38NH$_2$ antiserum diluted with buffer E were added, followed by reaction at 4°C for 16 hours. After the plate was washed with PBS, 100 μl of the HRP-labeled PACAP38NH$_2$ prepared in Example 2(1) described above [diluted 200 times with 0.02M phosphate buffer (pH 7.0) containing 2 mg/ml BSA and 0.15 M NaCl (buffer H)] was added to each well, followed by reaction at room temperature for 6 hours. After the reaction, the plate was washed with PBS, and then 100 µl of 0.1 M citrate buffer (pH 5.5) containing 0.2% o-phenylenediamine and 0.02% hydrogen peroxide was poured into each well, followed by reaction at room temperature for 10 minutes in order to measure the enzyme activity on the solid phase. After 100 µl of 4N sulfuric acid was added thereto to terminate the reaction, the absorption at 492 nm was measured by a plate reader (MTP-32, Corona). As a result, increases in anti-PACAP38 antibody titer were observed in 4 mice out of the 8 immunized mice.

**Example 3**

(1) Cell fusion

Mouse No.5, which exhibited a relatively high serum antibody titer in Example 2 above, was subjected to final immunization by intravenous injection of 200 µl of immunogen dissolved in 0.25 ml of saline. Four days after the final immunization, the spleen was extirpated from the mouse, compressed and filtered through stainless steel meshes, and suspended in Eagle's minimum essential medium (MEM), to yield a splenocyte suspension. BALB/C mouse-derived myeloma cells P3×63.Ag8.U1 (P3U1) were used as a cell fusion partner (Curr. Topics Microbiol. Immunol., 81: 1, 1978). Cell fusion was performed in accordance with an original method (Nature, 256: 495, 1957). That is, splenocytes and P3U1 were separately washed with serum-free MEM three times and mixed so that the ratio by number of the splenocytes and P3U1 was 5:1, and the mixed cells were precipitated by a centrifugation at 800 rpm for 15 minutes. After the supernatant was thoroughly removed, the precipitate was gently loosened, and 0.3 ml of 45% polyethylene glycol (PEG) 6000 (manufactured by Koch-light Ltd.) was added, and the mixture was allowed to stand in a warm water chamber at 37°C for 7 minutes to cause fusion. After the fusion, MEM was added to the cells at a rate of 2 ml per minute; after a total of 12 ml of MEM was added, the mixture was centrifuged at 600 rpm for 15 minutes, and the supernatant was removed. This cell precipitate was suspended in a GIT medium (Wako Pure Chemical) containing 10% fetal calf serum (GIT-10FCS) to obtain a P3U1 cell density of $2\times10^6$ cells per ml, and the suspension was inoculated to 120 wells of a 24-well multidish (manufactured by Linbro Company) at 1 ml per well. After the inoculation, the cells were cultured in a $CO_2$ incubator at 37°C under an atmosphere of 5% $CO_2$/95% air. After 24 hours, HAT selection culture was started by adding a GIT-10FCS medium containing HAT (hypoxanthine $1\times10^{-4}$ M, aminopterin $4\times10^{-7}$ M, thymidine $1.6\times10^{-3}$ M) (HAT medium) at 1 ml per well. The HAT selection culture was continued by discarding 1 ml of the old medium and thereafter adding 1 ml of HAT medium at 3, 6, and 9 days after the start of the cultivation. Colonies of hybridoma were observed 9 to 14 days after the cell fusion; when the culture medium turned to yellow (about $1\times10^6$ cells/ml of cell density), the supernatant was collected and antibody titer was measured.

(2) Hybridoma screening

Fifty microliter of buffer E and 50 µl of the hybridoma culture supernatant were added to the above-mentioned anti-mouse immunoglobulin antibody-bound microplate, and the reaction was carried out at room temperature for 6 hours. After the plate was washed with PBS, 100 µl of the HRP-labeled PACAP38NH$_2$ prepared in Example 2(1) above [diluted 200-fold with buffer H] was added, and the reaction was carried out at 4°C for 16 hours. Subsequently, after the plate was washed with PBS, the enzyme activity on the solid phase was measured by the method described in Example 2(2) above. The antibody titer was observed in 18 wells out of total of 120 wells showing hybridoma growth, when examined as described above.

(3) Cloning

Hybridomas of No.44, No.49, No.97, and No.113 wells out of the wells that showed anti-PACAP antibody positive were subjected to cloning by a limiting dilution method. That is, hybridomas were suspended in RPMI1640-20FCS to obtain a density of 1.5 cells/ml, and each suspension was dispensed to a 96-well microplate (manufactured by Nunc Company) at 0.2 ml per well. On this occasion, thymocytes isolated from BALB/C mouse were added to each well at $5\times10^5$ cells per well as feeder cells. About 1 week later, colony formation was observed, when the antibody titer in the culture supernatant was measured by the EIA method described in Example 2(2) above, 28 of the 30 clones of the hybridoma from No.44 well, 47 of the 50 clones of the hybridoma from No.49 well, 49 of the 50 clones of the hybridoma from No.97 well, and 48 of the 50 clones of the hybridoma from No.113 well produced the anti-PACAP antibody.

Taking note of some of these clones, namely, the clone PA-6N obtained from No.44-2 and the monoclonal antibody PA-6Na produced thereby, the clone PA-1N obtained from No.49-3 and the monoclonal antibody PA-1Na produced thereby, the clone PA-2C obtained from No.97-2 and the monoclonal antibody PA-2Ca produced thereby, and the clone PA-5N obtained from No.113-5 and the monoclonal antibody PA-5Na produced thereby, the following experiment was performed. Likewise, a cell fusion experiment was performed using splenocytes from another mouse immunized with the same immunogen, and taking note of the clone PA-1C obtained from No.28-12 and the monoclonal antibody PA-1Ca produced thereby and the clone PA-3N obtained from No.10-3 and the monoclonal antibody PA-3Na produced thereby, the following experiment was performed.

(4) Purification of Monoclonal Antibodies

The above-mentioned hybridoma was intraperitoneally injected to mouse (BALB/C) pre-treated with an intraperitoneally administration of mineral oil (0.5 ml) or the untreated mouse at 1-3 $\times$ $10^3$ cells/mouse, and ascites containing the antibody were collected 6-20 days later. The monoclonal antibodies were purified from the ascites by using a Protein-A column or a diethylaminoethyl (DEAE)-cellulose column. Namely, 6 ml of the ascites from the mice inoculated with PA-1N was diluted with the same amount of a binding buffer (1.5 M glycine buffer containing 3.5 M NaCl and 0.05% $NaN_3$ (pH 9.0)). The resulting solution was applied onto a Protein-A Sepharose column (Pharmacia) which had been pre-equilibrated with the binding buffer, and the specific antibody was eluted with an elution buffer (0.1 M citrate buffer containing 0.05% $NaN_3$ (pH 3.0)). By the above procedures, 28 mg of the specific antibody was obtained. Similarly, 23 mg of a specific antibody was obtained from 5 ml of the ascites from the mice inoculated with PA-5N, 13 mg of a specific antibody was obtained from 7.5 ml of the ascites from the mice inoculated with PA-6N, and 45 mg of a specific antibody was obtained from 14 ml of the ascites from the mice inoculated with PA-1C. On the other hand, a salt precipitation was applied to 20 ml of the ascites from the mice inoculated with PA-3N by adding saturated ammonium sulfate solution to a final concentration of 45%, which was followed by centrifugation (20,000 xg, 30 minutes). The precipitate fraction was dialyzed against 0.02 M borate buffer (pH 8) containing 0.15 M NaCl (BBS), and further dialyzed against 0.01 M phosphate buffer containing 0.01 M NaCl. The antibody fraction was applied on a DEAE cellulose column (DE-52, Wattman, 2.5 X 10 cm), and eluted by a 100 ml of linear concentration gradient (0.01 M-0.35 M) of NaCl. By the above procedures, 136 mg of a specific antibody was obtained. Similarly, 57 mg of a specific antibody was obtained from 7.5 ml of the ascites from the mice inoculated with PA-2C.

**Example 4** Determination of Class and Subclass of Monoclonal Antibody

One hundred microliter of 0.1 M carbonate buffer (pH 9.6) containing 5 $\mu$g/ml of $PACAP38NH_2$ was poured into each well of a 96-well microplate, and the microplate was allowed to stand at 4°C for 24 hours. The excess binding sites of the wells were blocked with Blockace according to the method described in Example 5. Thus, a $PACAP38NH_2$-bound plate was prepared. Then, each 100 $\mu$l of culture supernatants of PA-1N, PA-3N, PA-5N, PA-6N, PA-2C and PA-1C was added to the plate, followed by reaction at room temperature for 3 hours. Then, the class and subclass were examined by ELISA using an isotype typing kit (Mouse-Typer™ Sub-Isotyping Kit, Bio RAD). As a result, PA-1Na, PA-6Na, PA-2Ca and PA-1Ca belonged to IgGl, $\kappa$, PA-5Na belonged to IgG2a, $\kappa$ and PA-3Na belonged to IgG2b, $\kappa$.

**Example 5**

(1) Preparation of $F(ab')_2$ Fraction

PA-6Na was concentrated to 8 mg/500 $\mu$l by a Collodion bag (M&S Instruments Inc.), and then dialyzed against 0.1 M acetate buffer containing 0.1 M NaCl. To the resulting antibody solution was added 0.4 mg of pepsin (crystallized twice, Sigma), followed by reaction at 37°C for 16 hours. Then, the $F(ab')_2$ fraction was purified by an FPLC (Pharmacia) using a Superose 12 column equilibrated with 0.1 M phosphate buffer (pH 6.8). By a similar method, 0.445 mg of pepsin was added to 8.9 mg of PA-1Ca to prepare the $F(ab')_2$ fraction.

(2) Preparation of HRP-Labeled Anti-PACAP Monoclonal Antibodies

One milliliter of the PA-6Na $F(ab')_2$ fraction [2.2 mg (22 nmole)/ml] was mixed with 50 $\mu$l of a DMF solution containing GMBS(260 nmole), followed by reaction at room temperature for 40 minutes. The reaction solution was fractionated on a Sephadex G-25 column [1 X 30 cm, eluent: 0.1 M-phosphate buffer (pH 6.7)] to obtain 1.5 mg of a maleimide group-introduced $F(ab')_2$ fraction. With 1.5 mg of the maleimidated $F(ab')_2$ fraction was mixed with 5.5 mg of SH group-introduced HRP prepared by the method described in Example 2(1) above, and the reaction product was concentrated to about 0.3 ml by a collodion bag, followed by reaction at 4°C for 16 hours. The reaction solution was applied on an Ultrogel AcA34 column (10 mm $\phi$ x 40 mm) using 0.1 M phosphate buffer (PH 6.5) as an eluent to purify an $F(ab')_2$-HRP complex fraction. It was confirmed that 2.4 molecules of HRP were introduced per $F(ab')_2$ molecule by comparing the absorbance at 280 nm and 403 nm. In a similar manner, an $F(ab')_2$-HRP complex was prepared by using 2.9 mg of the PA-lCa F(ab')$_2$ fraction. Furthermore, 6.4 mg (43 nmoles) of the PA-2Ca purified fraction was maleimidated by adding 15-fold moles of GMBS. Then, the resulting product was reacted with SH group-introduced HRP in a similar manner to prepare the labeled antibody into which HRP was introduced in an amount of 2.4 molecules/molecule of IgG1.

**Example 6** Investigation of Region in Antigen Recognized by Each Monoclonal Antibody

(1) PA-lNa

Fifty microliter of a PA-1N culture supernatant diluted 50-fold with buffer H and 50 $\mu$l of a buffer H solution containing PACAP38NH$_2$, PACAP27NH$_2$, PACAP(4-27)OH, PACAP(1-13)OH, PACAP(14-38)NH$_2$, PACAP(31-38)NH$_2$ or VIP (SEQ ID NO:7) were added to the anti-mouse immunoglobulin antibody-bound microplate described in Example 2(2) above, followed by reaction at room temperature for 2 hours. Then, 50 $\mu$l of HRP-labeled PACAP38NH$_2$ obtained in Example 2(1) above (diluted 100-fold with buffer H) was added thereto, followed by reaction at 4˚C for 16 hours. After the reaction, the plate was washed with PBS, and then the enzyme activity on the solid phase was measured by the method described in Example 2(2) above. The results are shown in FIG. 1(a). PA-lNa reacted with PACAP38NH$_2$, PACAP27NH$_2$, PACAP(1-13)OH and PACAP(4-27)OH, but did not react with PACAP(14-38)NH$_2$ and PACAP(31-38) NH$_2$. PA-lNa did not react with VIP either (the cross reactivity was 0.1% or less (to PACAP38NH$_2$)). These results reveal that the antibody recognizes the N-terminal portion of PACAP38NH$_2$.

(2) PA-3Na

A competitive EIA using PA-3Na (50-fold dilution of a PA-3N culture supernatant) was carried out by the method described in the above (1). The results are shown in FIG. 1(b). PA-3Na reacted with PACAP38NH$_2$, PACAP27NH$_2$, PACAP(1-13)OH and PACAP(4-27)OH, but did not react with PACAP(14-38)NH$_2$ and PACAP(31-38)NH$_2$ (the cross reactivity was 0.1% or less (to PACAP38NH$_2$)). PA-3Na showed cross reactivity of 1% with VIP (to PACAP38NH$_2$). These results reveal that the antibody recognizes the N-terminal portion of PACAP38NH$_2$.

(3) PA-5Na

A competitive EIA using PA-5Na (70-fold dilution of a PA-5N culture supernatant) was carried out by the method described in the above (1). The results are shown in FIG. 1(c). PA-5Na reacted with PACAP38NH$_2$, PACAP27NH$_2$, PACAP(1-13)OH and PACAP(4-27)OH, but did not react with PACAP(14-38)NH$_2$ and PACAP(31-38)NH$_2$. PA-5Na did not react with VIP either (the cross reactivity was 0.1% or less (to PACAP38NH$_2$)). These results reveal that the antibody recognizes the N-terminal portion of PACAP38NH$_2$. PA-1Na was different from PA-5Na in cross reactivity to PACAP (1-13)OH (in comparison with PACAP38NH$_2$), and the cross reactivity of the former was at least 10 times stronger than that of the latter.

(4) PA-6Na

A competitive EIA using PA-6Na (40-fold dilution of a PA-6N culture supernatant) was carried out by the method described in the above (1). The results are shown in FIG. 1(d). PA-6Na reacted with PACAP38NH$_2$, PACAP27NH$_2$ and PACAP(4-27)OH, but did not react with PACAP(1-13)OH, PACAP(14-38)NH$_2$ and PACAP(31-38)NH$_2$. PA-6Na did not react with VIP either (the cross reactivity was 0.1% or less (to PACAP38NH$_2$)). These results reveal that the antibody recognizes the region from the N-terminal portion to the central portion of PACAP38NH$_2$.

(5) PA-2Ca

A competitive EIA using PA-2Ca (340-fold dilution of a PA-2C culture supernatant) was carried out by the method described in the above (1). The results are shown in FIG. 1(e). PA-2Ca reacted with PACAP38NH$_2$ and PACAP (14-38)NH$_2$, but did not react with PACAP27NH$_2$, PACAP(4-27)OH PACAP(1-13)OH and PACAP(31-38)NH$_2$. PA-2Ca did not react with VIP either (the cross reactivity was 0.1% or less to PACAP38NH$_2$). These results reveal that the antibody recognizes the region from the C-terminal portion to the central portion of PACAP38NH$_2$.

(6) PA-1Ca

A competitive EIA using PA-1Ca (35-fold dilution of a PA-1C culture supernatant) was carried out by the method described in the above (1). The results are shown in FIG. 1(f). PA-1Ca reacted with PACAP38NH$_2$, PACAP(14-38)NH$_2$ and PACAP (31-38) NH$_2$, but did not react with PACAP27NH$_2$, PACAP(4-27)OH and PACAP(1-13)OH. PA-1Ca did not react with VIP either (the cross reactivity was 0.1% or less (to PACAP38NH$_2$)). These results reveal that the antibody recognizes the C-terminal portion of PACAP38NH$_2$.

Based on the results shown above, the region in antigen recognized by the above-described six kinds of anti-PACAP monoclonal antibodies are summarized in FIG. 2.

**Example 7:** Evaluation of neutralizing activities of anti-PACAP monoclonal antibodies

Rat adrenal pheochromocytoma cell line, PC-12h [supplied by Dr. Hatanaka (then) of the Institute for Protein Research, Osaka University; Brain Res., 222(2): 225-33, 1981] were seeded into a collagen-coated 48-well multiwell plate (manufactured by Sumitomo Bakelite Co., Ltd.) at $5\times10^4$ cells/well, and cultured in Dulbecco's modified Eagle's medium (DMEM) containing 10% FCS for 7 to 10 days. The medium in the plate was replaced with Hanks' balanced salt solution (HBSS) containing 0.05% BSA, and the cells were cultured for 30 minutes. Then, PACAP38NH$_2$ (final concentration 2 nM) that had been previously reacted with each of the above-described six kinds of anti-PACAP monoclonal antibodies (final concentration 2, 20 or 200 nM) at 4˚C for 1 hour, was added to the plate. After the cells were further cultured for 2 hours, the cAMP concentration in the culture supernatant was measured using a cAMP assay kit (manufactured by Amersham Company). The results are shown in FIG. 3. It was found that two antibodies (PA-6Na and PA-1Ca) out of the six kinds of anti-PACAP monoclonal antibodies had no neutralizing activity against PACAP38NH$_2$.

**Example 8:** Evaluation of suppressive effect of anti-PACAP38 non-neutralizing antibodies on PACAP degradation by DPP-IV

(1) Suppressive effect on PACAP degradation by endogenous DPP-IV from CaCo-2 cells

Whether or not anti-PACAP38 non-neutralizing antibodies suppress PACAP degradation by DPP-IV was investigated using DPP-IV (dipeptidyl peptidase IV) expressed in CaCo-2 cells (human colon adenocarcinoma-derived cells).

After a CaCo-2 cell membrane fraction was placed in Tris-EDTA-CHAPS buffer [20 mM Tris-HCl (pH 7.5) containing 5 mM EDTA, 0.1% BSA and 0.05% CHAPS] and diluted stepwise, PACAP38 was added at a final concentration of $1.0\times10^{-8}$ M in the presence or absence of $1.3\times10^{-7}$ M of PA-6Na, and the mixture was incubated at 37˚C for 4.5 hours (experiment 1). To evaluate the non-selective adsorption of PACAP38 to the CaCo-2 cell membrane fraction, $4\times10^{-7}$ M of a DPP-IV inhibitor (IC$_{50}$=5 nM) was added in advance, and the mixture was incubated under the same conditions as experiment 1 (experiment 2). After $4\times10^{-7}$ M of the above-described DPP-IV inhibitor was added to the sample of experiment 1 to stop the reaction, the reaction mixtures of experiments 1 and 2 were diluted 10-fold with Tris-EDTA-Digitonin buffer [20 mM Tris-HCl (pH 7.5) containing 5 mM EDTA, 0.1% BSA, 0.05% Digitonin, 0.5 mM PMSF, 10 μg/ml Pepstatin A, 20 μg/ml Leupeptin, and 4 μg/ml E-64], and 100 pM of a PACAP receptor [purified using the method described in Ohtaki, T. et al.: J Biol Chem 273, 15464-73. (1998) with partial modification] and 100 pM of $^{125}$I-PACAP27 (Ohtaki T et al. Biochem. Biophys. Res. Commun. 171, 838-844) were added thereto. After incubation at 25˚C for 75 minutes, the reaction mixture was applied to suction filtration using a polyethylenimine-treated GF/F glass filter (Whatman), and the radioactivity of the $^{125}$I-PACAP27 trapped on the filter was detected using a γ counter. The measurements were performed in triplicate. The results are shown in FIG. 4. The stronger residual radioactivity shows larger amount of $^{125}$I-PACAP27 bound to the receptor, indicating decrease in the residual amount of the previously added PACAP38 in sample. From this Figure, it was found that DPP-IV exhibited concentration-dependent PACAP38 degradation in the absence of PA-6Na, whereas PA-6Na nearly completely suppressed the degradation of PACAP38 in the range of DPP-IV concentrations examined.

(2) Suppressive effect on degradation by a recombinant DPP-IV

The same kind of experiment was performed under the same reaction conditions as with the use of CaCo-2 but using semipurified recombinant DPP-IV in place of the CaCo-2 membrane fraction. The use of the recombinant DPP-IV lessened the non-selective binding, which was problematic during the evaluation of the PACAP38 degradation with the CaCo-2 membrane fraction, and improved the substrate degradation activity (FIG. 5, -□-). Irrespective of the presence or absence of a DPP-IV inhibitor, PA-6Na suppressed the degradation of PACAP38 by DPP-IV in the DPP-IV concentration range from 1- to 100-fold dilution (FIG. 5, -■- and -O-). That is, a suppression effect of PA-6Na on PACAP38 degradation by DPP-IV was clearly detected without previously adding the DPP-IV inhibitor.

**Example 9**: Preparation of an immunogen comprising GLP-1(7-36) amide

Four milligram of GLP-1(7-36)amide (manufactured by AnaSpec Company) and 7.2 mg of BSA were dissolved in 2 ml of 0.1M phosphate buffer (pH 6.8), and 2 ml of glutaraldehyde, previously diluted to 0.1% in 0.1M phosphate buffer (pH 6.8) was gently added thereto drop by drop, and the reaction was carried out under ice cooling for 5 hours. After the reaction mixture was dialyzed against calcium- and magnesium-free Dulbecco's phosphate buffered saline (D-PBS(-)), the dialysate was preserved in divided portions at -80°C, and this was used as the immunogen.

**Example 10:** Measurement of anti-GLP-1 antibody concentrations (enzyme immunoassay)

An anti-mouse immunoglobulin goat antibody (IgG fraction, manufactured by Jackson ImmunoResearch Laboratories Inc.) was diluted to a concentration of 5 $\mu$g/ml in 0.01 M phosphate buffer (pH 8.0) containing 0.01 M NaCl, this was dispensed to a 96-well half-area plate (manufactured by Corning Inc.) at 50 $\mu$l per well, and the antibody was adsorbed at 4°C overnight. After this solution was removed, a D-PBS(-) containing 25% BlockAce (manufactured by Dainippon Pharmaceutical Co., Ltd.) was added at 100 $\mu$l per well, and the plate was allowed to stand at 37°C for 1 hour.
After this plate was washed with a D-PBS containing 0.05% Tween 20 (PBS-T), 50 $\mu$l portion of an antiserum, previously serially diluted with a D-PBS containing 0.1% Tween 20, was added, and the reaction was carried out at 37°C for 1 hour. After the plate was washed with PBS-T, a biotin-labeled GLP-1 [GLP-1 (7-36)-Lys (Biotin), amide] (manufactured by AnaSpec Inc.), previously diluted to 10 ng/ml with D-PBS(-) containing 0.1% Tween 20, was dispensed at 50 $\mu$l per well, and the reaction was carried out at 37°C for 1 hour. After the plate was washed with PBS-T, horseradish peroxidase (HRP)-labeled streptavidin (manufactured by Jackson ImmunoResearch Laboratories Inc.), previously diluted to 0.3 $\mu$g/ml with D-PBS(-) containing 0.1% Tween, was dispensed at 50 $\mu$l per well, and the reaction was carried out at room temperature for 30 minutes. After the plate was further washed with PBS-T, an HRP substrate (TMB Peroxidase EIA Substrate Kit, manufactured by Bio-Rad Laboratories, Inc.) was dispensed at 50 $\mu$l per well to develop a color; after 1 N sulfuric acid was dispensed at 50 $\mu$l per well to stop the reaction, absorbance at 450 nm was measured using a plate reader (Multiscan; Labosystems Japan Co.).

**Example 11**

(1) Immunization with GLP-1 (7-36) amide complex

Ten BALB/c mice (13-week-old, female) were subcutaneously immunized with an emulsion prepared by mixing the GLP-1(7-36)amide/BSA complex solution prepared in Example 9 and Freund's complete adjuvant in a 1:1 ratio, by volume at 50 $\mu$g/animal. In the second immunization and thereafter, an emulsion prepared with the GLP-1(7-36) amide/BSA complex and Freund's incomplete adjuvant was given at 2-week intervals. One week after the third immunization, final immunization was performed by intravenously administering the above-described immunogen dissolved in 100 $\mu$l of saline to individuals showing high serum antibody titers in the ELISA described in Example 10.

(2) Obtainment of anti-GLP-1 antibody-producing hybridomas

Three days after the final immunization, the spleen was extirpated from the mouse, and about $10^8$ splenocytes were obtained via centrifugal washing procedure. After the splenocytes and mouse myeloma cells P3X63.Ag8.U1 (P3U1) were mixed at a 5:1 cell count ratio, the cells were fused by using polyethylene glycol 1500 (manufactured by Roche Diagnostics) in accordance with the method of Kohler and Milstein (Nature, 256: 495, 1957). The fused cells were resuspended in an IH medium [a 1:1 mixed medium of IMDM (manufactured by Invitrogen Co.) and Ham's F-12 (manufactured by Invitrogen Co.)] containing 10% fetal bovine serum (IH-FCS), and seeded into a 96-well multiple well plate (manufactured by Corning Inc.) at $2\times10^4$ cells/100 $\mu$l/well based on P3U1 cell, and cultured in a $CO_2$ incubator at 37°C under 5% $CO_2$ atmosphere overnight. The following day, an IH-FCS containing hypoxanthine, aminopterin and thymidine (HAT medium) was added to each well at 100 $\mu$l/well, and selection culture was performed in a $CO_2$ incubator at 37°C under 5% $CO_2$ atmosphere. For the HAT selection culture, 3/4 of the culture supernatant from each well was exchanged with a fresh HAT medium 4 days and 7 days after the cell fusion, and the culture was continued. The culture supernatants from the wells showing hybridoma growth from 8 days to 2 weeks after the cell fusion were subjected to the ELISA described in Example 10 and 15 kinds of anti-GLP-1(7-36) monoclonal antibody-producing hybridomas [GLIT1-175(1) 18, GLIT1-238(1) 6, GLIT1-254(1)6, GLIT1-256(1)31, GLIT1-464(1)15, GLIT1-492(1)2, GLIT2-105(1)6, GLIT2-130(1) 36, GLIT2-197(1)66, GLIT2-234(2)65, GLIT2-329(1)24, GLIT2-357(1) 10, GLIT2-806(1) 4, GLIT2-851(1) 11, GLIT2-863 (1)35] were obtained, and cloned cell lines were established by a limiting dilution method.

(3) Preparation of purified anti-GLP-1 monoclonal antibodies

The 15 kinds of anti-GLP-1 monoclonal antibody-producing hybridomas obtained above were cultured in an IH medium containing 10% Ultra low IgG FCS (manufactured by Invitrogen Co.) in a $CO_2$ incubator at 37°C under 5%

$CO_2$ atmosphere, and the culture supernatants were collected by centrifugation and subjected to Protein A column chromatography to yield purified antibodies. The subclasses of these purified antibodies were determined using the IsoStrip Mouse Monoclonal Antibody Isotyping Kit (manufactured by Roche Diagnostics). These anti-GLP-1 antibodies exhibited high binding activities of $EC_{50}$: $1\times10^{-9}$ to $1\times10^{-11}$ M, against a biotin-labeled GLP-1(7-36) amide, in the ELISA described in Example 10. Also, in the same ELISA, a given concentration of each of the various anti-GLP-1 antibodies was trapped onto an anti-mouse immunoglobulin goat antibody-coated plate, after which a given concentration (10 ng/mL) of a biotin-labeled GLP-1(7-36)amide and a 0.003- to 300-fold molar amount of GLP-1(7-36)amide (manufactured by AnaSpec Company) or GLP-1(9-36) amide (manufactured by Phoenix Pharmaceuticals Company) were added, and the binding inhibition of the labeled GLP-1(7-36)amide were examined; as a result, all antibodies were found to be antibodies specifically recognizing the N-terminal region of GLP-1, which were strongly inhibited by GLP-1(7-36)amide but were not inhibited by GLP-1(9-36)amide. The above results are summarized in Table 1.

[Table 1] Biotin-labeled GLP-1(7-36)amide binding inhibitory activities ($IC_{50}$) of various anti-GLP-1 antibodies

| | Biotin-labeled GLP-1(7-36) amide binding inhibitory activity (IC50(M)) | |
| --- | --- | --- |
| Name of antibody clone | GLP-1(7-36)amide | GLP-1(9-36)amide |
| GLIT1-175(1)18 | 2.5E-09 | >1.4E-07 |
| GLIT1-238(1)6 | 1.9E-09 | >1.4E-07 |
| GLIT1-254(1)6 | 1.4E-09 | 5.1E-07 |
| GLIT1-256(1)31 | 2.4E-09 | >1.4E-07 |
| GLIT1-464(1)15 | 1.6E-09 | >1.4E-07 |
| GLIT1-492(1)2 | 1.5E-09 | 5.8E-07 |
| GLIT2-105(1)6 | 2.4E-09 | >1.4E-07 |
| GLIT2-130(1)36 | 2.0E-09 | >1.4E-07 |
| GLIT2-197(1)66 | 4.0E-09 | >1.4E-07 |
| GLIT2-234(2)65 | 3.0E-09 | >1.4E-07 |
| GLIT2-329(1)24 | 3.6E-09 | 2.1E-06 |
| GLIT2-357(1)10 | 5.8E-09 | >1.4E-07 |
| GLIT2-806(1)4 | 9.0E-09 | 7.6E-07 |
| GLIT2-851(1)11 | 2.0E-09 | >1.4E-07 |
| GLIT2-863(1)35 | 2.2E-09 | >1.4E-07 |

**Example 12:** Selection of anti-GLP-1 non-neutralizing monoclonal antibody

To select a non-neutralizing antibody that does not inhibit the binding of GLP-1 to GLP-1 receptor, out of the 15 kinds of anti-GLP-1 monoclonal antibodies prepared in Example 11, the following two kinds of assays measuring GLP-1 activity were performed.

(1). GLP-1 receptor reporter gene assay

A CHO cell line stably expressing GLP-1 receptor on the cell membrane surface and transfected with the Multiple Response Element (MRE)/cAMP Response Element (CRE)-luciferase gene, were suspended in Ham's F-12 medium (manufactured by Invitrogen Co.) containing 10% fetal bovine serum, 1 $\mu$g/ml blastocidin, 500 $\mu$g/ml geneticin, and 50 mg/ml gentamycin, and the cell suspension was seeded into a 96-well white plate (manufactured by Corning Coster Co.) at $1\times10^4$ cells/well, and cultured using a $CO_2$ incubator at 37°C under 5% $CO_2$ atmosphere for 2 days. After the medium was removed by aspiration, 100 $\mu$l of a reaction mixture of GLP-1(7-36)amide (2 nM) and a 1- to 300-fold molar amount of purified anti-GLP-1 monoclonal antibody[diluted with Ham's F-12 (manufactured by Invitrogen Co.)], pre-incubated at room temperature for 1 hour, was added, and the cells were cultured in a $CO_2$ incubator at 37°C under 5% $CO_2$ atmosphere for 5.5 hours. After the medium was removed by aspiration, a 1:1 mixture of PicaGene LT-FR (manufactured by Toyo Ink MFG Co., Ltd.) and Hanks' balanced salt solution (HBSS) was added to each well at 40 $\mu$l/ well. The reaction was carried out at room temperature for 20 minutes, after which transcriptional activity on MRE/CRE was quantified with luciferase activity as the index, by using the Wallac 1420 ARVOMX Multilabel counter (manufactured by PerkinElmer Japan Company).

(2) GLP-1/GLP-1 receptor binding inhibition assay

A cell membrane fraction wherein the GLP-1 receptor was overexpressed was prepared as described below. That is, a recombinant baculovirus solution was prepared from the pFastBac™ plasmid (manufactured by Invitrogen Lifetechnologies Company) inserted with the human GLP-1 receptor gene by a conventional method, and the virus was infected to an insect cell line (Sf9) to transiently express the GLP-1 receptor. These infected cells were collected by a centrifugation and disrupted with Polytron in the presence of a surfactant, after which the cell debris was removed by a centrifugation at 600 x g, and the supernatant was ultracentrifuged at 100,000 x g to yield a membrane fraction containing the GLP-1 receptor.

Two hundreds picomolar of $^{125}$I-labeled GLP-1 (IM323, manufactured by Amersham Bioscience) and a 1- to 1,000-fold molar amount of anti-GLP-1 antibody were mixed and incubated at 37˚C for 1 hour, after which the above-described GLP-1 receptor membrane fraction was added, and the mixture was incubated at room temperature for 75 minutes, after which B/F separation was performed using a polyethylenimine-treated glass filter (GF/F, manufactured by Watman), and the amount of $^{125}$I-labeled GLP-1 bound to the GLP-1 receptor was quantified using a $\gamma$ counter.

The results of the above experiment (1) and (2) are summarized in Table 2. Antibodies that did not inhibit the binding of GLP-1 to GLP-1 receptor even when added at a 1,000-fold molar amount to GLP-1(7-36)amide, and that did not neutralize GLP-1 activity in the GLP-1 receptor reporter gene assay even when added at a 300-fold molar amount (see FIG. 6 and FIG. 7) were designated as anti-GLP-1 non-neutralizing antibodies. Out of the two kinds of anti-GLP-1 non-neutralizing antibodies obtained (Table 2), GLIT2-329(1)24, which showed better physical stability, was selected as the anti-GLP-1 non-neutralizing antibody and subjected to the experiment described below.

[Table 2] Results of GLP-1 receptor reporter gene assay and GLP-1/GLP-1 receptor binding inhibition assay of anti-GLP-1 antibodies

| Name of antibody clone | GLP-1 receptor reporter gene assay | GLP-1/GLP-1 receptor binding inhibition assay |
|---|---|---|
| GLIT1-175(1)18 | - | ++ |
| GLIT1-238(1)6 | - | ++ |
| GLIT1-254(1)6 | +++ | +++ |
| GLIT1-256(1)31 | ++ | + |
| GLIT1-464(1)15 | +++ | binds to GF |
| GLIT1-492(1)2 | ++ | +++ |
| GLIT2-105(1)6 | - | + |
| GLIT2-130(1)36 | - | + |
| GLIT2-197(1)66 | - | + |
| GLIT2-234(2)65 | - | + |
| GLIT2-329(1)24 | - | - |
| GLIT2-357(1)10 | - | + |
| GLIT2-806(1)4 | - | - |
| GLIT2-851(1)11 | - | + |
| GLIT2-863(1)35 | + | +++ |
| +: Positive for neutralization activity, -: Negative for neutralization activity, GF: Glass filter | | |

**Example 13**: Suppressive effect of anti-GLP-1 non-neutralizing monoclonal antibody (GLIT2-329(1)24) on GLP-1(7-36) amide degradation by DPP-IV

The suppressive effect of the anti-GLP-1 non-neutralizing monoclonal antibody (GLIT2-329(1)24) selected in Example 12 on GLP-1(7-36)amide degradation by DPP-IV was examined by ELISA. GLP-1(7-36)amide (final concentration 10 µg/ml) was added to a PBS-T-diluted mixture of anti-GLP-1 monoclonal antibody (GLIT2-329(1)24; final concentration 5 mg/ml) and Dipeptidyl peptidase IV (DPP-IV) (R&D Systems Company; final concentration 200 ng/ml), and the reaction was carried out at 37°C for 12 hours. After thermally treated at 95°C for 5 minutes, the reaction mixture was centrifuged at 1800 rpm, and the residual amount of GLP-1(7-36)amide in the supernatant was measured by the method described below. An anti-GLP-1 antibody (GLIT1-254(1)6)that binds to the N-terminal region of GLP-1(7-36) amide but does not bind to GLP-1(9-36)amide was diluted to a concentration of 0.15 µg/ml in D-PBS(-), this was dispensed to a 96-well half-area plate (manufactured by Corning Company) at 50 µl per well, and the antibody was adsorbed at 4°C overnight. After the solution was removed, 100 µl of a D-PBS(-) containing 25% BlockAce (manufactured by Dainippon Pharmaceutical Co., Ltd.) was added to each well, and the plate was allowed to stand at room temperature for 1 hour. After the plate was washed with PBS-T, the above-mentioned reaction mixture, previously serially diluted with a D-PBS(-) containing 0.1% Tween 20, and biotin-labeled GLP-1(7-36)amide, previously diluted to 5 ng/ml with a D-PBS(-) containing 0.1% Tween 20, were dispensed each at 25 µl per well, and the reaction was carried out at 37°C for 2 hours. After the plate was washed with PBS-T, HRP-labeled streptavidin (manufactured by Jackson ImmunoResearch Laboratories Inc.), previously diluted to 0.3 µg/ml with a D-PBS(-) containing 0.1% Tween, was dispensed at 50 µl per well, and the reaction was carried out at room temperature for 30 minutes. After the plate was further washed with PBS-T, an HRP substrate (TMB Peroxidase EIA Substrate Kit, manufactured by Bio-Rad Laboratories Inc.) was dispensed at 50 µl per well to develop a color, and 1 N sulfuric acid was dispensed at 50 µl per well to stop the reaction, after which absorbance at 450 nm was measured using a plate reader (Multiscan; Labosystems Japan Co.).

In the absence of antibody (None) and in the presence of anti-erythropoietin antibody (Anti-EPO Ab), More than 90% of GLP-1(7-36)amide was degraded by DPP-IV; whereas in the presence of anti-GLP-1 non-neutralizing monoclonal antibody (GLIT2-329(1)24), only about 10% of GLP-1(7-36)amide was degraded; it was found that this anti-GLP-1 non-neutralizing monoclonal antibody is capable of suppressing the degradation of GLP-1(7-36)amide by DPP-IV (FIG. 8).

**Example 14:** *In vivo* administration experiment of anti-GLP-1 non-neutralizing monoclonal antibody

(1) Administration of antibody into rat peritoneal cavity

Saline, an anti-GLP-1 non-neutralizing monoclonal antibody (GLIT2-329(1)24) or control mouse IgG (ChromoPure mouse IgG, whole molecule; manufactured by Jackson ImmunoResearch Laboratories Inc.) was intraperitoneally administered to Wister fatty rats (female) in satiated state at 20 mg/kg: 1 day later, whole blood was drawn from the abdominal aorta, and a DPP-IV inhibitor (DPP4, LINCO Research) was immediately added thereto. Plasma was prepared by centrifugation and cryopreserved.

(2) Measurement of rat plasma GLP-1(7-36)amide concentrations

The rat plasma concentration of GLP-1(7-36) amide was measured using a GLP-1(7-36)amide assay kit (manufactured by Linco Company) after the pretreatment described below. That is, to a 0.75-mL plasma sample taken from each rat in each administration group described above, an equal volume of Buffer A (1% TFA in 99% distilled water) was added. After the reaction at 4°C for 5 minutes, it was centrifuged and the supernatant was collected. The supernatant was passed through a C18 column (Sep-column containing 200 mg of C18, manufactured by Peninsula Laboratories Inc.) equilibrated with Buffer A, and the column was washed with Buffer A, followed by elution with Buffer B (60% acetonitrile and 1% TFA in 39% distilled water). The eluate was concentrated to dryness under reduced pressure, the residue was dissolved again in 375 µL of the Assay Buffer attached to the above-described ELISA kit, and thermally treated (95°C, 5 minutes), after which it was centrifuged, and the supernatant was subjected to the above-described ELISA kit to measure the GLP-1(7-36)amide concentration. According to the results of an addition-recovery experiment wherein GLP-1(7-36)amide was added to rat plasma and the GLP-1(7-36)amide concentration was measured after the same pretreatment as abovementioned, the recovery of GLP-1(7-36)amide through the pretreatment was 30%. Rat plasma GLP-1(7-36)amide concentrations as corrected by this value are shown in FIG. 9.

The plasma concentration of active form GLP-1(7-36) in saline-administered satiated Wister Fatty rats at 1 day after administration was 4.7 ± 0.6 pM, and that of active form GLP-1(7-36) in the mouse IgG administration group was 7.1 ± 1.2 pM. On the other hand, the plasma concentration of active form GLP-1(7-36) in the anti-GLP-1 non-neutralizing antibody (GLIT2-329(1)24) administration group was 14.1 ± 4.0 pM, the concentration significantly higher than that of in the mouse IgG administration group as the control (p<0.05). This result shows that it is possible to extend the blood half-life of active form GLP-1(7-36) in a living rat, and to raise the blood level thereof by administering the anti-GLP-1 non-neutralizing antibody.

According to a report describing pre-administration of a DPP-IV inhibitor to an experimental system wherein Zucker fatty rats were loaded with glucose administered from the duodenum, the maximum plasma concentration of active form GLP-1 was about 10 pM at the time when the compound promoted insulin secretion and suppressed blood glucose elevation (Diabetes, 51, I461-1469, 2002). From this result, it is considered that the active form GLP-1(7-36) concentration observed in the anti-GLP-1 non-neutralizing antibody (GLIT2-329(1)24) administration group in this Example, 14.1 $\pm$ 4.0 pM, reached in its pharmacologically effective range.

**Industrial Applicability**

When the agent for improving the blood stability of the present invention is administered to an animal, the non-neutralizing antibody as the active ingredient thereof binds to an endogenous ligand and stabilizes the same, to raise the blood ligand concentration and/or extend the blood half-life of the ligand, and hence to enhance the receptor activity-regulatory action of the ligand; therefore, the agent of the present invention is useful for the prophylaxis and treatment for a disease involved by an abnormality in the activity of the receptor. In particular, the agent for improving the blood stability of the present invention is useful for a disease involved by an endogenous ligand having a very short blood half-life, such as a peptidic compound, or a disease involved by a receptor for which no agonist is easy to obtain, and the like.

Also, because the agent for improving the blood stability of the present invention exhibits its effect at an amount of antibody sufficient to obtain the desired blood ligand concentration, it enables a remarkable reduction in clinical dose compared with existing antibody medicines and enables the provision of a safer and inexpensive preparation. Therefore, the agent for improving the blood stability of the present invention contributes to medical cost reductions and significantly contributes to the expansion of the coverage of indications of antibody medicines.

This application is based on a patent application No. 2004-098595 filed in Japan (filing date: March 30, 2004), the contents of which are incorporated in full herein by this reference.

## Claims

1. An agent for improving the blood stability of a mammalian endogenous ligand, which comprises an antibody that has an affinity to the endogenous ligand but does not neutralize the same substantially.

2. The agent of claim 1, wherein the improved blood stability of the endogenous ligand results in the enhancement of receptor activity-regulatory action thereof.

3. The agent of claim 1, wherein the neutralizing activity of the antibody is about 80% or less.

4. The agent of claim 1, wherein the blood concentration of the endogenous ligand becomes about twice or more compared to the case where the antibody is not administered.

5. The agent of claim 1, wherein the blood half-life of the complex of the endogenous ligand and the antibody is about twice or more as that of the endogenous ligand alone.

6. The agent of claim 1, wherein the blood half-life of the free endogenous ligand is about one week or less.

7. The agent of claim 1, wherein the endogenous ligand is a peptidic compound.

8. The agent of claim 7, wherein the endogenous ligand is one against a G protein-coupled receptor.

9. The agent of claim 8, wherein the endogenous ligand is one belonging to secretin/glucagon super family.

10. The agent of claim 9, wherein the endogenous ligand is selected from the group consisting of GLP-1, calcitonin, PACAP, VIP and analogs thereof.

11. The agent of claim 8, wherein the endogenous ligand is selected from the group consisting of LHRH, metastin, GPR7/GPR8 ligand, MSH, ghrelin, apelin and analogs thereof.

12. The agent of claim 7, wherein the endogenous ligand is selected from the group consisting of EPO, TPO, insulin, interferon, growth hormone, GM-CSF, leptin, adiponectin and analogs thereof.

13. The agent of claim 7, wherein the endogenous ligand is selected from the group consisting of ANP, BNP, CNP, betacellulin, betacellulin-δ4, adrenomedullin and analogs thereof.

14. The agent of claim 1, which is for the prophylaxis and/or treatment of a disease in which an increased blood concentration and/or a prolonged blood half-life of the endogenous ligand are/is effective for the prophylaxis and/or treatment thereof.

15. The agent of claim 14, wherein the disease is selected from the group consisting of metabolic disease, bone and joint disease, cardiovascular disease, cranial nerve disease, infectious disease, cancer, blood disorder, urologic disease, infertility/erectile dysfunction, deficient growth and immunodeficiency.

16. A method for the prophylaxis and/or treatment of a disease in a mammal, wherein an increased blood concentration and/or a prolonged blood half-life of an endogenous ligand are/is effective for the prophylaxis and/or treatment of the disease, which method comprises administering to the mammal an effective amount of an antibody that has an affinity to the endogenous ligand but does not neutralize the same substantially, without administering a compound the same as or substantially the same as the endogenous ligand, so as to increase the blood stability of the endogenous ligand, thereby enhancing a receptor activity-regulatory action of the ligand.

17. A use of an antibody that has an affinity for an endogenous ligand but does not neutralize the same substantially for the manufacture of an agent for the prophylaxis and/or treatment of a disease in which an increased blood concentration and/or a prolonged blood half-life of the endogenous ligand are/is effective for the prophylaxis and/or treatment thereof.

FIG. 1

(a) PA-1Na

(b) PA-3Na

(c) PA-5Na

(d) PA-6Na

(e) PA-2Ca

(f) PA-1Ca

# FIG. 2

PA-1Na

PA-3Na    PA-6Na    PA-2Ca PA-1Ca

PA-5Na

# FIG. 3

## FIG. 4

# FIG. 5

# FIG. 6A

# FIG. 6B

FIG. 7

EP 1 731 168 A1

# FIG. 8

# FIG. 9

GLP-1 concentration (pM)

Saline    Mouse IgG antibody    GLIT2-329(1)24

* : P<0.05

**INTERNATIONAL SEARCH REPORT**

| International application No. |
| --- |
| PCT/JP2005/006576 |

A. CLASSIFICATION OF SUBJECT MATTER
Int.Cl⁷ A61K39/395, 47/48, A61P3/00, 5/02, 5/06, 5/18, 5/48, 7/00, 9/00,
15/00, 15/10, 15/18, 19/08, 25/00, 31/00, 35/00, 37/04

According to International Patent Classification (IPC) or to both national classification and IPC

B. FIELDS SEARCHED

Minimum documentation searched (classification system followed by classification symbols)
Int.Cl⁷ A61K39/395, 47/48

Documentation searched other than minimum documentation to the extent that such documents are included in the fields searched

| | | | |
| --- | --- | --- | --- |
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
REGISTRY/CA/MEDLINE/EMBASE/BIOSIS(STN), JSTPlus/JMEDPlus(JOIS)

C. DOCUMENTS CONSIDERED TO BE RELEVANT

| Category* | Citation of document, with indication, where appropriate, of the relevant passages | Relevant to claim No. |
| --- | --- | --- |
| X | JP 60-502104 A (HYBRITECH INC.), 05 December, 1985 (05.12.85), Claims; details of test & EP 467416 A1 & WO 85/974 A1 | 1-15,17 |
| X | JP 1-138461 A (HYBRISENS LTD.), 31 May, 1989 (31.05.89), Claims & EP 298654 A2 & US 5686579 A | 1-15,17 |
| X | JP 4-120025 A (Chugai Pharmaceutical Co., Ltd.), 21 April, 1992 (21.04.92), Claims; page 2, upper right column (Family: none) | 1-15,17 |

| ☒ | Further documents are listed in the continuation of Box C. | ☐ | See patent family annex. |
| --- | --- | --- | --- |

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 26 April, 2005 (26.04.05) | 17 May, 2005 (17.05.05) |

| Name and mailing address of the ISA/ | Authorized officer |
| --- | --- |
| Japanese Patent Office | |
| Facsimile No. | Telephone No. |

Form PCT/ISA/210 (second sheet) (January 2004)

**EP 1 731 168 A1**

INTERNATIONAL SEARCH REPORT

International application No.

PCT/JP2005/006576

C (Continuation).   DOCUMENTS CONSIDERED TO BE RELEVANT

| Category* | Citation of document, with indication, where appropriate, of the relevant passages | Relevant to claim No. |
|---|---|---|
| X | JP 3-133378 A  (Ivan E. Modrovich),<br>06 June, 1991 (06.06.91),<br>Claims; examples<br>& US 5660978 A | 1-15,17 |
| X | JP 10-504708 A  (Beringu Diagnostics GmbH),<br>12 May, 1998 (12.05.98),<br>Claims<br>& WO 96/284 A1 | 1-15,17 |

Form PCT/ISA/210 (continuation of second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0179494 A
- JP 60502104 A
- US 6331415 B
- US 5225539 A
- US 5585089 A
- US 5693761 A
- US 5693762 A
- JP 5227970 A
- US 5939598 A
- US 5545806 A

### Non-patent literature cited in the description

- **J. SAMBROOK et al.** Molecular Cloning. Cold Spring Harbor Lab. Press, 1989
- **M. BODANSZKY ; M.A. ONDETTI.** Peptide Synthesis. Interscience Publishers, 1966
- **SCHROEDER ; LUEBKE.** *The Peptide,* 1965
- *Bio/Technology,* 1991, vol. 9, 88-89
- *Nat. Genet.,* 1997, vol. 15, 146-156
- *Nat. Biotechnol.,* 1996, vol. 14, 845-851
- *Nat. Biotechnol.,* 2000, vol. 18, 1086-1090
- *Nat. Biotechnol.,* 2002, vol. 20, 889-894
- **HOLT et al.** *Curr. Opin. Biotechnol.,* 2000, vol. 11, 445-449
- *J. Biol. Chem.,* 1999, vol. 274, 18218-18230
- *J. Mol. Biol.,* 1991, vol. 222, 581-597
- *Nat. Biotechnol.,* 1996, vol. 14, 309-314
- *Annu. Rev. Immunol.,* 1994, vol. 12, 433-455
- *J. Biol. Chem.,* 1999
- *Proc. Natl. Acad. Sci. USA,* 2000, vol. 14, 7969-7974
- *J. Mol. Biol.,* 2000, vol. 296, 57-86
- *Nat. Biotechnol.,* 2000, vol. 18, 852
- *Proc. Natl. Acad. Sci. USA,* 1995, vol. 92, 3938
- *J. Immunol. Methods,* 2003, vol. 272, 219-233
- *Curr. Topics Microbiol. Immunol.,* 1978, vol. 81, 1
- *Nature,* 1957, vol. 256, 495
- **DR. HATANAKA.** Institute for Protein Research. *Brain Res.,* 1981, vol. 222 (2), 225-33
- **OHTAKI, T. et al.** *J Biol Chem,* 1998, vol. 273, 15464-73
- **OHTAKI T et al.** *Biochem. Biophys. Res. Commun.,* vol. 171, 838-844

SEQUENCE LISTING

<110> Takeda Pharmaceutical Company Limited

<120> Antibody Medicines

<130> 09754

<150> JP2004-098595
<151> 2004-03-30

<160> 7

<170> PatentIn version 3.2

<210> 1
<211> 38
<212> PRT
<213> Homo sapiens

<220>
<221> MOD_RES
<222> (38)..(38)
<223> AMIDATION

<400> 1

His Ser Asp Gly Ile Phe Thr Asp Ser Tyr Ser Arg Tyr Arg Lys Gln
1               5                   10                  15

Met Ala Val Lys Lys Tyr Leu Ala Ala Val Leu Gly Lys Arg Tyr Lys
                20                  25                  30

Gln Arg Val Lys Asn Lys
            35

<210> 2
<211> 27
<212> PRT
<213> Homo sapiens

<220>
<221> MOD_RES
<222> (27)..(27)
<223> AMIDATION

<400> 2

His Ser Asp Gly Ile Phe Thr Asp Ser Tyr Ser Arg Tyr Arg Lys Gln
1               5                   10                  15

Met Ala Val Lys Lys Tyr Leu Ala Ala Val Leu
                20                  25

<210> 3
<211> 25
<212> PRT
<213> Artificial Sequence

<220>
<223> Synthetic peptide having amino acid sequence identical to partial
      amino acid sequence of human PACAP38.

<220>
<221> MOD_RES
<222> (25)..(25)
<223> AMIDATION

```
<400>  3

Arg Lys Gln Met Ala Val Lys Lys Tyr Leu Ala Ala Val Leu Gly Lys
1               5                   10              15


Arg Tyr Lys Gln Arg Val Lys Asn Lys
            20                  25



<210>  4
<211>  13
<212>  PRT
<213>  Artificial Sequence

<220>
<223>  Synthetic peptide having amino acid sequence identical to partial
       amino acid sequence of human PACAP.

<400>  4

His Ser Asp Gly Ile Phe Thr Asp Ser Tyr Ser Arg Tyr
1               5                   10


<210>  5
<211>  24
<212>  PRT
<213>  Artificial Sequence

<220>
<223>  Synthetic peptide having amino acid sequence identical to partial
       amino acid sequence of human PACAP.

<400>  5

Gly Ile Phe Thr Asp Ser Tyr Ser Arg Tyr Arg Lys Gln Met Ala Val
1               5                   10              15


Lys Lys Tyr Leu Ala Ala Val Leu
            20


<210>  6
<211>  8
<212>  PRT
<213>  Artificial Sequence

<220>
<223>  Synthetic peptide having amino acid sequence identical to partial
       amino acid sequence of human PACAP38.


<220>
<221>  MOD_RES
<222>  (8)..(8)
<223>  AMIDATION

<400>  6

Tyr Lys Gln Arg Val Lys Asn Lys
1               5


<210>  7
<211>  28
<212>  PRT
<213>  Homo sapiens


<220>
<221>  MOD_RES
<222>  (28)..(28)
<223>  AMIDATION
```

```
<400>  7

His Ser Asp Ala Val Phe Thr Asp Asn Tyr Thr Arg Leu Arg Lys Gln
1               5                   10                  15


Met Ala Val Lys Lys Tyr Leu Asn Ser Ile Leu Asn
            20                  25
```